# FASCICULE DE BREVET EUROPEEN

(11) **EP 2 890 364 B1**
(45) Date de publication et mention de la délivrance du brevet: **10.01.2018**
(21) Numéro de dépôt: 13748032.3
(22) Date de dépôt: 12.08.2013
(51) Int. Cl.: A61K 9/107, A61K 9/51, A61K 48/00

(54) **FORMULATION POUR LA DELIVRANCE DE SEQUENCES NUCLEOTIDIQUES SUSCEPTIBLES DE MODULER DES MECANISMES ENDOGENES D'ARN INTERFERENTS**
FORMULIERUNG ZUR VERABREICHUNG VON NUKLEOTIDSEQUENZEN ZUR MODULIERUNG ENDOGENER INTERFERIERENDER RNA-MECHANISMEN
FORMULATION FOR THE DELIVERY OF NUCLEOTIDE SEQUENCES THAT CAN MODULATE ENDOGENOUS INTERFERING RNA MECHANISMS

(30) Priorité: 30.08.2012 FR 1258115
(43) Date de publication de la demande: 08.07.2015
(73) Titulaire: Commissariat à l'Energie Atomique et aux Energies Alternatives, 75015 Paris (FR)
(72) Inventeur: NAVARRO Y GARCIA, Fabrice, 38600 Fontaine (FR); BRUNIAUX, Jonathan, 38000 Grenoble (FR); GIDROL, Xavier, 06570 Saint Paul De Vence (FR); SULPICE, Eric, 38330 Biviers (FR); TEXIER-NOGUES, Isabelle, 38000 Grenoble (FR)
(74) Mandataire: Lavoix
(86) Numéro de dépôt international: PCT/EP2013/066821
(87) Numéro de publication internationale: WO 2014/032953

(56) Documents cités:
- WO-A1-2010/018223
- WO-A2-2012/006380

## Description

La présente invention concerne une formulation de type nanoémulsion utile pour la délivrance de séquences nucléotidiques susceptibles de moduler des mécanismes endogènes d'ARN interférents.

Le mécanisme d'interférence par ARN est un mécanisme d'inhibition post-transcriptionnelle de l'expression des gènes et repose sur de petits ARN interférents double brin qui dégradent spécifiquement un ARNm cible et inhibent très sélectivement et spécifiquement l'expression d'une protéine. Ce mécanisme se déroule dans le cytoplasme et met en jeu un complexe multi-protéique nommé le complexe RISC (en anglais RNA-induced silencing complex).

Depuis la découverte de ce mécanisme naturel, des séquences nucléotidiques susceptibles de moduler des mécanismes endogènes d'ARN interférents sont utilisées dans de nombreux laboratoires académiques et/ou pharmaceutiques comme outils de recherche dans plusieurs disciplines de la biologie. Par exemple, les séquences nucléotidiques susceptibles de moduler des mécanismes endogènes d'ARN interférents sont utilisées pour révéler de nouvelles cibles thérapeutiques dans de nombreuses pathologies. En inhibant très sélectivement et spécifiquement l'expression de telle ou telle protéine, on peut ainsi mettre en relief le rôle qu'elle joue dans le processus de développement de la pathologie. Néanmoins, l'introduction de matériel génétique exogène dans la cellule (la transfection) requiert une méthode adaptée physique, chimique ou biologique, pour permettre à ces séquences nucléotidiques susceptibles de moduler des mécanismes endogènes d'ARN interférence, qui sont généralement de grosses molécules (>10 kDa) et sont chargées négativement, de traverser la membrane plasmique chargée négativement qui constitue une véritable barrière.

Parmi les méthodes physiques, on peut citer l'électroporation ou la génération de pores dans la membrane par ultrasons. Parmi les méthodes biologiques, on peut citer le recours à des systèmes spécialisés dans la délivrance d'acides nucléiques tels que les virus. Parmi les méthodes chimiques, on peut citer l'utilisation :
- de lipoplexes (complexes formés entre des acides nucléiques et des lipides cationiques), par exemple la lipofectamine® 2000,
- de polyplexes (complexes formés entre des acides nucléiques et des polymères cationiques, par exemple le polyéthylèneimine PEI),
- de complexes formés entre des acides nucléiques et des dendrimères, par exemple les dendrimères à base de poly(amidoamine) PANAM,
- de lipopolyplexes (complexes formés entre des acides nucléiques, des liposomes et des polymères), par exemple les SNALPs (« stable nucleic acid-lipid particles » en anglais), qui correspondent à des mélanges de lipides cationiques et auxiliaires formant des liposomes, stabilisés par des polymères de type polyethylene-glycol (PEG),
- de complexes formés entre des acides nucléiques et des nanoparticules polymériques à base de poly(DL-lactide-co-glycolide) PLGA.

A la connaissance des inventeurs, seul Park et al. (Molecular Pharmaceutics 5, 4, 622-631, 2008) décrit l'utilisation de nanoparticules lipidiques pour la délivrance de siARN. Les nanoparticules lipidiques comprennent du cholestérol, de la 1,2-Dioléoyl-sn-Glycéro-3-Phosphoéthanolamine (DOPE) et un dérivé de siARN et ont été utilisées pour la transfection et l'extinction de la protéine GFP. Toutefois, pour éviter la fuite du siARN hors des nanoparticules, ceux-ci ont été greffés de manière covalente à celles-ci. Les siARN ont en effet été liés de manière covalente à une chaîne poly(oxyde d'éthylène) qui s'intercale dans les nanoparticules et permet de fixer les siARN. De plus, un marqueur fluorescent (agent d'imagerie) a également été greffé de manière covalente aux siARN pour le suivi de la transfection. Or, toute modification chimique du siARN est susceptible de le dénaturer. De plus, la technique de Park et al. nécessite de modifier chimiquement chaque siARN utilisé, ce qui est long et coûteux. Il existe donc un besoin de fournir un système de délivrance de séquences nucléotidiques susceptibles de moduler des mécanismes endogènes d'ARN interférence dans lequel ceux-ci ne sont pas modifiés chimiquement.

De plus, la demande de brevet WO 2010/018223 décrit l'utilisation d'une formulation sous forme de nanoémulsion, comprenant une phase aqueuse continue et au moins une phase dispersée comprenant un lipide amphiphile, un lipide solubilisant, un agent thérapeutique et un co-tensioactif comprenant au moins une chaîne composée de motifs d'oxyde d'alkylène, pour la délivrance d'un agent thérapeutique amphiphile ou lipophile. L'agent thérapeutique peut notamment être un siARN. Toutefois, les siARN étant des composés hydrophiles, ils se situeraient dans la phase aqueuse continue de la nanoémulsion, et non dans la phase dispersée. La formulation décrite dans cette demande n'est donc pas adaptée pour la délivrance de séquences nucléotidiques susceptibles de moduler des mécanismes endogènes d'ARN interférents. De plus, les présents inventeurs ont mis en évidence que toutes les formulations décrites dans WO 2010/018223, et en particulier celles décrites dans les exemples, même en y intégrant des tensioactifs cationiques, ne sont pas adaptées pour la présente application, car le rendement de complexation est très faible, et la transfection ne serait pas efficace. En effet, comme expliqué ci-après, seules des formulations comprenant des proportions spécifiques de composants permettent la délivrance de séquences nucléotidiques susceptibles de moduler des mécanismes endogènes d'ARN interférents.

Par ailleurs, des systèmes de délivrance d'acide désoxyribonucléique (ADN) à base d'émulsion huile dans l'eau ou de gouttelettes lipidiques sont connues. Dans ces systèmes, les gouttelettes lipidiques comprennent des tensioactifs cationiques, grâce auxquels l'ADN se lie aux gouttelettes par des interactions électrostatiques.

Par exemple, Del Pozo-Rodriguez et al. (International Journal of Pharmaceutics, 385, 2010, 157-162) ont observé une présence de fluorescence dans le foie et la rate de souris auxquelles ont été administrées par voie intraveineuse des nanoparticules lipidiques comprenant de l'ADN plasmidique nu (pADN) de la protéine fluorescente EGFP, du Precirol® ATO 5, du Tween 80, du chlorure de 1,2-dioleoyl-3-triméthylammoniumpropane (DOTAP) (tensioactif cationique) pour transfecter in vitro des cellules HEK293 et in vivo après administration à des souris.

Tabatt et al. (European Journal of Pharmaceutics and Biopharmaceutics 57, 2004, 155) décrit l'utilisation de nanoparticules lipidiques solides comprenant du Compritol ATO 888 ou du cetylpalmitate, du Tween 80, du Span 85, un tensioactif cationique choisi parmi le chlorure d' alkyldimethylbenzylammonium (BA), le bromure de tétradécyltriméthylammonium (CTAB), le chlorure de hexadécylpyridinium (CPC), le bromure de diméthyldioctadécylammonium (DDAB), le chlorure de N,N-di-(b-stéaroyléthyl)-N,N-diméthyl-ammonium (Esterquat 1, EQ 1) et le chlorure de 8, N-[1-(2,3-dioléoyloxy)propyl]-N,N,Ntriméthylammonium (DOTAP) pour transfecter in vitro des cellules COS-1.

Dans ces deux articles, du Tween 80 a été utilisé. Or, celui-ci présente une certaine toxicité vis-à-vis des cellules. De plus, le Tween 80 comprend des chaînes poly(oxyde d'éthylène) comprenant 20 unités d'oxyde d'éthylène. Comme expliqué ci-après, les inventeurs ont mis en évidence qu'un tensioactif ayant des chaînes poly(oxyde d'éthylène) plus longues (au moins 25 unités d'oxyde d'éthylène) est plus facile à formuler, est plus stable, et permet de protéger plus efficacement les séquences nucléotidiques comprises dans la formulation vis-à-vis du milieu extérieur.

Enfin, les séquences nucléotidiques susceptibles de moduler des mécanismes endogènes d'ARN interférence, en particulier le siARN, sont généralement moins stables que l'ADN. De plus, les activités biologiques d'ADN et de siARN sont très différentes, ce qui induit que les systèmes de délivrance adaptés pour l'ADN ne sont pas nécessairement les mêmes que ceux adaptés pour la délivrance de siARN. En particulier, même si dans les deux cas, un prérequis est un système de délivrance permettant de réaliser des transfections efficaces, cela n'est pas suffisant pour un système de délivrance de siARN qui ne sera intéressant que s'il permet d'éteindre le gène.

Le développement de nouvelles formulations permettant la délivrance in vivo et in vitro de séquences nucléotidiques susceptibles de moduler des mécanismes endogènes d'ARN interférence est donc nécessaire.

### [Formulation]

A cet effet, selon un premier objet, la présente invention fournit une formulation sous forme de nanoémulsion, comprenant une phase aqueuse continue et au moins une phase dispersée.

L'invention fournit une formulation comprenant une séquence nucléotidique susceptible de moduler des mécanismes endogènes d'ARN interférents qui permet la délivrance desdites séquences.

Elle fournit également une formulation, dite « prémix », exempte de séquence nucléotidique susceptible de moduler des mécanismes endogènes d'ARN interférence, qui permet la préparation d'une formulation comprenant une séquence nucléotidique susceptible de moduler des mécanismes endogènes d'ARN interférents par complexation d'une séquence nucléotidique susceptible de moduler des mécanismes endogènes d'interférence par ARN avec cette formulation « prémix ». Cette formulation « prémix » peut donc avantageusement être complexée avec une séquence nucléotidique susceptible de moduler des mécanismes endogènes d'ARN interférence adaptée selon l'utilisation désirée de la formulation finale.

### [Formulation de type prémix]

Ainsi, selon un premier objet, la présente invention fournit une formulation sous forme de nanoémulsion, comprenant une phase aqueuse continue et au moins une phase dispersée, et comprenant :
- au moins 5% molaire de lipide amphiphile,
- de 15 à 70% molaire d'au moins un tensioactif cationique comprenant :
   - au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, et
      - un poly(oxyde de propylène), et
   - au moins un groupe hydrophile comprenant au moins un groupe cationique choisi parmi :
      - un groupe alkyle linéaire ou ramifié comprenant de 1 à 12 atomes de carbone et interrompu et/ou substitué par au moins un groupe cationique, et
      - un groupe polymérique hydrophile comprenant au moins un groupe cationique, et
- de 10% à 55% molaire d'un co-tensioactif comprenant au moins une chaîne poly(oxyde d'éthylène) comprenant au moins 25 unités d'oxyde d'éthylène,
- un lipide solubilisant comprenant au moins un glycéride d'acides gras,
- éventuellement un lipide fusogène,
où les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

Comme expliqué ci-après, les : lipide amphiphile / tensioactif cationique / co-tensioactif / éventuel lipide fusogène sont les principaux composants de la couronne des gouttelettes de la formulation « premix ».

L'émulsion est donc une émulsion de type huile dans l'eau. Elle peut être simple ou multiple, notamment en comportant dans la phase dispersée une seconde phase aqueuse. De préférence, elle est simple.

L'invention repose sur la découverte inattendue que la formulation décrite ci-dessus peut être utilisée comme agent de transfection et/ou pour la délivrance in vitro et in vivo de séquences nucléotidiques susceptibles de moduler des mécanismes endogènes d'ARN interférence. La formulation présente avantageusement une bonne biodisponibilité et peut permettre de limiter la dégradation des séquences nucléotidiques susceptibles de moduler des mécanismes endogènes d'ARN interférents généralement observée avec d'autres systèmes de délivrance, notamment la dégradation par des protéines.

De plus, la formulation est avantageusement stable : elle peut être stockée plusieurs heures sans qu'aucune dégradation ne soit observée.

### • Tensioactif cationique

La formulation selon l'invention comprend un tensioactif cationique comprenant :
- au moins un groupe lipophile choisi parmi :
   - un groupe R représentant une chaîne hydrocarbonée linéaire comprenant de 11 à 23 atomes de carbone,
   - un ester ou un amide d'acides gras comprenant de 12 à 24 atomes de carbone et de phosphatidyléthanolamine, tel que le distéaryl phosphatidyléthanolamine (DSPE), et
   - un poly(oxyde de propylène), et
- au moins un groupe hydrophile comprenant au moins un groupe cationique choisi parmi :
   - un groupe alkyle linéaire ou ramifié comprenant de 1 à 12 atomes de carbone et interrompu et/ou substitué par au moins un groupe cationique, et
   - un groupe polymérique hydrophile comprenant au moins un groupe cationique, ledit groupe polymérique étant notamment choisi parmi :
      - un poly(oxyde d'éthylène) comprenant typiquement de 3 à 500 unités oxyde d'éthylène, de préférence de 20 à 200 unités oxyde d'éthylène, et comprenant au moins un groupe cationique.
      - un polysaccharide, tel que du dextrane, de la cellulose ou du chitosan, ayant notamment des masses moléculaires comprises entre 0,5 et 20 kDa, par exemple entre 1 et 12 kDa,
      - un polyamine, tel qu'un chitosan ou une polylysine, ayant notamment des masses moléculaires comprises entre 0,5 et 20 kDa, par exemple entre 1 et 12 kDa.

Par «ester ou amide d'acides gras comprenant de 12 à 24 atomes de carbone et de phosphatidyléthanolamine», on entend un groupe de formule : 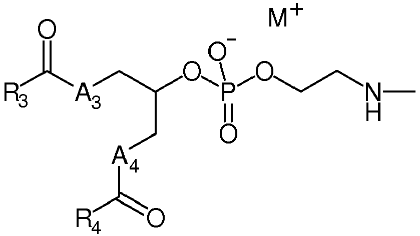 dans laquelle
- R₃ et R₄ représentent indépendamment une chaîne hydrocarbonée linéaire comprenant de 11 à 23 atomes de carbone,
- A₃ et A₄ représentent O ou NH, et
- M représente H ou un cation.

Les groupes cationiques du tensioactif cationique sont typiquement :
- des oniums choisis parmi les groupes ammonium, imidazolium, pyridinium, pyrrolidinium, pipéridinium, phosphonium ou sulfonium, ou
- des complexes métalliques entre un radical d'un groupe organique chélatant mono- ou multi-dentate, par exemple la phénantroline, la pyridine, l'acide éthylène diamine tétraacétique (EDTA), l'acide diéthylène triamine penta acétique (DTPA), les porphyrines, les phtalocyanines, les clorines, les bactériochlorines complexé avec un cation inorganique, tel que Ca²⁺, Al³⁺, Ni⁺, Zn²⁺, Fe²⁺, Fe³⁺ ou Cu²⁺,
- les groupes ammonium, notamment -⁺NMe₃, -⁺NHMe₂, -⁺NH₂Me et -⁺NH₃, en particulier -⁺NH₃, étant particulièrement préféré.

Bien sûr, des anions sont associés au(x) groupe(s) cationique(s) pour que la formulation soit électriquement neutre. La nature des anions n'est pas limitée. A titre illustratif, on peut citer les halogénures, notamment le chlorure ou le bromure, ou le trifluoroacétate.

Dans le tensioactif cationique, la nature du groupe de liaison liant le(s) groupe(s) lipophile(s) au(x) groupe(s) hydrophile(s) comprenant au moins un groupe cationique n'est pas limitée. Des exemples de groupes de liaison sont fournis ci-dessous (groupe L).

Dans un mode de réalisation, le tensioactif cationique a la formule (A) suivante :

[(Lipo)ₗ-L-(Hydro)ₕ]ⁿ⁺, (n/m) [A]^{m-} (A)

dans laquelle:
- l et h représentent des nombres entiers indépendamment compris entre 1 et 4,
- n est un nombre entier supérieur ou égal à 1, généralement compris entre 1 et 50,
- Lipo représente un groupe lipophile tel que défini ci-dessus,
- Hydro représente un groupe hydrophile tel que défini ci-dessus comprenant au moins un groupe cationique,
- L représente un groupe de liaison,
- A représente un anion,
- m est un nombre entier représentant la charge de l'anion,
- n est un nombre entier représentant la charge du cation [(Lipo)ₗ-L-(Hydro)ₕ].

Dans la formule (A) susmentionnée, de préférence L est tel que :
- lorsque l et h représentent 1, L est un groupe de liaison divalent choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH, -O-PO(OH)-O- ou un radical divalent cyclique de 5 à 6 atomes,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents,
- lorsque l'un des groupes l ou h représente 1, et l'autre représente 2, L est un groupe trivalent choisi parmi un groupe phosphate OP-(O-)₃, un groupe dérivé du glycérol de formule -O-CH₂-CH-(O-)CH₂-O- et un radical trivalent cyclique de 5 à 6 atomes,
- pour les autres valeurs de l et h, L est un radical multivalent cyclique de 5 à 6 atomes.

De manière particulièrement préférée, L est tel que :
- lorsque l et h représentent 1, L est un groupe de liaison divalent choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH ou -O-PO(OH)-O-,
- lorsque l'un des groupes l ou h représente 1, et l'autre représente 2, L est un groupe trivalent choisi parmi un groupe phosphate OP-(O-)₃ et un groupe dérivé du glycérol de formule -O-CH₂-CH-(O-)CH₂-O-.

Dans la formule (A) susmentionnée, l et h représentent de préférence indépendamment 1 ou 2.

Selon une première alternative, le groupe hydrophile du tensioactif cationique est un groupe alkyle linéaire ou ramifié comprenant de 1 à 12 atomes de carbone et interrompu et/ou substitué par au moins un groupe cationique. Comme exemple de tels tensioactifs cationiques, on peut citer:
1) (Lipo)-(CH₂)ₘ₁-NR₃₀R₃₁R₃₂, où Lipo est un groupe lipophile tel que défini ci-dessus, m1 représente 1 ou 2 et R₃₀, R₃₁ et R₃₂ représentent indépendamment H, Me ou -CH₂-CH₂-OH,
2) 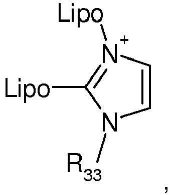 où chaque Lipo est indépendamment un groupe lipophile tel que défini ci-dessus, et R33 représente H, Me ou -CH₂-CH₂-OH,
3) 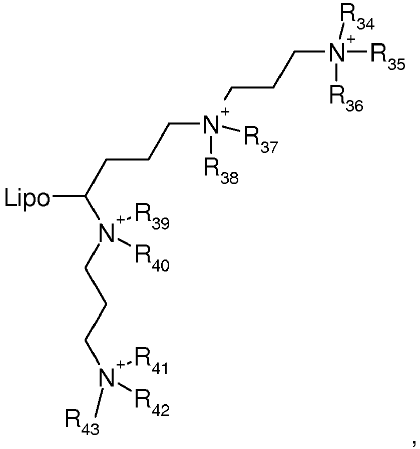 où Lipo est un groupe lipophile tel que défini ci-dessus, et R₃₄, R₃₅, R₃₆, R₃₇, R₃₈, R₃₉, R₄₀, R₄₁, R₄₂ et R₄₃ représentent indépendamment H, Me ou -CH₂-CH₂-OH.

Dans un mode de réalisation, le tensioactif cationique est choisi parmi :
- le *N*[1-(2,3-dioléyloxy) propyl]-*N,N,N-*triméthylammonium (DOTMA),
- le 1,2-dioleyl-3-trimethylamonium-propane (DOTAP),
- le *N*-(2-hydroxyethyl)-*N,N-*dimethyl-2,3-bis(tetradecyloxy-1-propananium) (DMRIE),
- le 1-[2-(oleoyloxy)ethyl]-2-oleyl-3-(2-hydroxyethyl)imidazolinium (DOTIM), et
- le dioctadecylamidoglycylspermine (DOGS) (sous forme protonée),
et est de préférence le 1,2-dioleyl-3-trimethylamonium-propane (DOTAP).

Selon une deuxième alternative, le groupe hydrophile du tensioactif cationique est un groupe polymérique hydrophile comprenant au moins un groupe cationique.

Lorsque le groupe hydrophile du tensioactif cationique est polymérique, le(s) groupe(s) cationique(s) peu(ven)t être un(des) groupe(s) terminal(aux) ou pendant(s). Par exemple :
- lorsque groupe polymérique hydrophile est un poly(oxyde d'éthylène), le(s) groupe(s) cationique(s) est(sont) généralement situé(s) sur un groupe terminal à l'extrémité de la chaîne poly(oxyde d'éthylène).
- lorsque le groupe polymérique hydrophile est du dextrane ou de la cellulose, le(s) groupe(s) cationiques est(sont) généralement situé(s) sur un groupe terminal à l'extrémité de la chaîne polysaccharidique.
- lorsque le groupe polymérique hydrophile est du chitosan, le(s) groupe(s) cationiques est(sont) généralement un(des) groupe(s) pendant(s), en particulier des groupes - NH₃⁺ présents en milieu acide sur le chitosan.

Dans un mode de réalisation, le(s) groupe(s) cationique(s) est(sont) un(des) groupe(s) terminal(aux). En effet, les groupes pendants de tensioactifs anioniques adjacents en surface des gouttelettes de la phase dispersée se repoussent par interactions électrostatiques et, par conséquent, les formulations comprenant des tensioactifs cationiques dont le groupe Hydro comprend des groupes pendants sont généralement moins stables.

Dans un autre mode de réalisation, le(s) groupe(s) cationique(s) est(sont) un(des) groupe(s) pendant(s). Il est avantageusement possible d'utiliser un tensioactif cationique dont le groupe hydrophile comprend plusieurs groupes cationiques pendants, et donc d'obtenir une formulation plus chargée positivement, et qui permettra d'autant mieux la complexation d'espèces négatives comme les séquences nucléotidiques susceptibles de moduler des mécanismes endogènes d'ARN interférents.

Le groupe polymérique hydrophile préféré est un radical d'un poly(oxyde d'éthylène) comprenant typiquement de 3 à 500 unités oxyde d'éthylène, de préférence de 20 à 200 unités oxyde d'éthylène, et comprenant au moins un groupe cationique.

Ainsi, dans un mode de réalisation, le tensioactif cationique a l'une des formules suivantes : 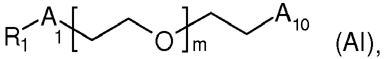 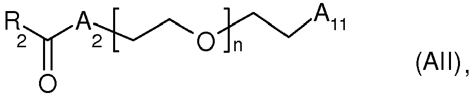 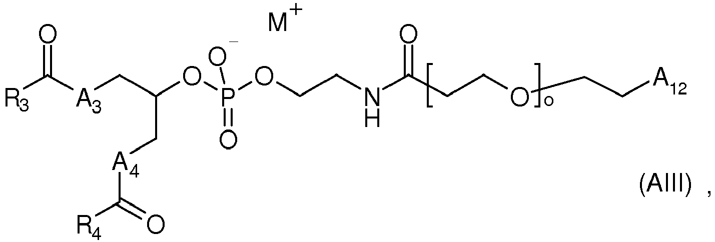 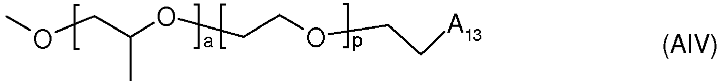 dans lesquelles :
- R₁, R₂, R₃ et R₄ représentent indépendamment une chaîne hydrocarbonée linéaire comprenant de 11 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation,
- A₁₀, A₁₁, A₁₂ et A₁₃ représentent indépendamment un groupe -⁺NR₂₀R₂₁R₂₂, dans lequel R₂₀, R₂₁ et R₂₂ représentent indépendamment H, Me ou -CH₂-CH₂-OH.

Dans un mode de réalisation, dans la formule (All), A₁₁ représente -⁺NH₃ et le tensioactif cationique a la formule suivante : 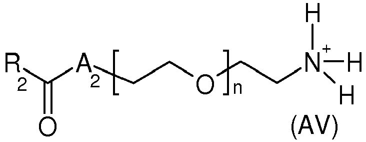 dans laquelle A₂, R₂ et n sont tels que définis ci-dessus. De préférence, dans la formule (All), R₂ représente C₁₇H₃₅.

Sans vouloir être liés à une théorie particulière, la présence du groupe polymérique hydrophile permettrait :
- de stabiliser la formulation, et
- de protéger les séquences nucléotidiques susceptibles de moduler des mécanismes endogènes d'ARN interférents localisés à la surface des gouttelettes des protéines du milieu dans lequel la formulation est administrée/utilisée, et donc la dégradation des séquences nucléotidiques susceptibles de moduler des mécanismes endogènes d'ARN interférents par ces protéines.

Selon une troisième alternative, la formulation selon l'invention comprend au moins deux tensioactifs cationiques, dont :
- l'un est choisi parmi :
   - le *N*[1-(2,3-dioléyloxy) propyl]-*N,N,N-*triméthylammonium (DOTMA),
   - le 1,2-dioleyl-3-trimethylamonium-propane (DOTAP),
   - le *N*-(2-hydroxyethyl)-*N,N-*dimethyl-2,3-bis(tetradecyloxy-1-propananium) (DMRIE),
   - le 1-[2-(oleoyloxy)ethyl]-2-oleyl-3-(2-hydroxyethyl)imidazolinium chloride (DOTIM), et
   - le dioctadecylamidoglycylspermine (DOGS),
   et est de préférence le 1,2-dioleyl-3-trimethylamonium-propane (DOTAP), et
- l'autre est un tensioactif cationique comprenant :
   - au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, tel que le distéaryl phosphatidyléthanolamine (DSPE), et
      - un poly(oxyde de propylène), et
   - un groupe polymérique hydrophile comprenant au moins un groupe cationique, ledit groupe polymérique étant choisi parmi :
      - un poly(oxyde d'éthylène) comprenant typiquement de 3 à 500 unités oxyde d'éthylène, de préférence de 20 à 200 unités oxyde d'éthylène, et comprenant au moins un groupe cationique,
      - un polysaccharide, tel que du dextrane, de la cellulose ou du chitosan,
      - un polyamine, tel qu'un chitosan ou une polylysine,
et est de préférence un poly(oxyde d'éthylène) comprenant au moins un groupe cationique.

Dans un mode de réalisation, la formulation selon l'invention comprend, à titre de tensioactifs cationiques :
- du 1,2-dioleyl-3-trimethylamonium-propane, et
- un tensioactif cationique comprenant :
   - au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, tel que le distéaryl phosphatidyléthanolamine (DSPE), et
   - un poly(oxyde d'éthylène) comprenant typiquement de 3 à 500 unités oxyde d'éthylène, de préférence de 20 à 200 unités oxyde d'éthylène, et comprenant au moins un groupe cationique.

Dans un mode de réalisation, la formulation selon l'invention comprend, à titre de tensioactifs cationiques :
- du 1,2-dioleyl-3-trimethylamonium-propane, et
- un composé de formule (All) telle que définie ci-dessus, notamment de formule (AV).

Le tensioactif cationique se situe dans la couronne des gouttelettes de la formulation. Il se lie par interactions électrostatiques aux séquences nucléotidiques susceptibles de moduler des mécanismes endogènes d'ARN interférence et permet de maintenir les siARN à la surface des gouttelettes.

La formulation comprend de 15 à 70% molaire d'au moins un tensioactif cationique par rapport à l'ensemble (lipide amphiphile / tensioactif cationique / co-tensioactif / éventuel lipide fusogène). En dessous de 15%, la formulation ne comprend pas assez de charges positives et la complexation ultérieure de la formulation « prémix » avec les séquences nucléotidiques (chargées négativement) est insuffisante. Au-delà de 70%, les formulations ne sont pas stables, et ne peuvent généralement même pas être formulées (la formation de la nanoémulsion n'est pas possible car les gouttelettes coalescent pour former deux phases), et les gouttelettes deviennent généralement toxiques pour les cellules.

Ces proportions sont particulièrement adaptées pour obtenir une complexation efficace des séquences nucléotidiques susceptibles de moduler des mécanismes endogènes d'ARN interférents, et donc une bonne délivrance et/ou transfection.

### • Lipide fusogène (« helper lipid » en anglais)

Dans un mode de réalisation, la formulation selon l'invention comprend un lipide fusogène, qui est susceptible de faciliter la libération cytosolique par déstabilisation de la membrane endosomale, dit lipide « helper ». De préférence, ce lipide est le dioléylphosphatidyléthanolamine (DOPE).

Ce lipide permet de favoriser l'échappement endosomal des gouttelettes de la formulation selon l'invention, et donc des séquences nucléotidiques susceptibles de moduler des mécanismes endogènes d'interférence par ARN qu'elles contiennent, et améliore généralement l'efficacité d'extinction du gène d'intérêt.

Le lipide susceptible de faciliter la libération cytosolique par déstabilisation de la membrane endosomale se situe dans la couronne des gouttelettes de la formulation.

### • lipide amphiphile

La formulation comprend au moins un lipide amphiphile, qui se situe dans la couronne des gouttelettes de la formulation.

Afin de former une nanoémulsion stable, il est nécessaire d'inclure dans la composition au moins un lipide amphiphile à titre de tensioactif. La nature amphiphile du tensioactif assure la stabilisation des gouttelettes d'huile au sein de la phase continue aqueuse. En dessous de 5% molaire de lipide amphiphile par rapport à l'ensemble (lipide amphiphile / tensioactif cationique / co-tensioactif / éventuel lipide fusogène), les formulations ne sont pas stables, et ne peuvent généralement même pas être formulées (la formation de la nanoémulsion n'est pas possible car les gouttelettes coalescent pour former deux phases).

Généralement, la formulation comprend de 5 à 85% molaire, de préférence de 5 à 75% molaire, en particulier de 5 à 50% molaire et tout particulièrement de 8 à 30% molaire de lipide amphiphile par rapport à l'ensemble (lipide amphiphile / tensioactif cationique / co-tensioactif / éventuel lipide fusogène).

La quantité de lipide amphiphile contribue avantageusement à contrôler la taille de la phase dispersée de la nanoémulsion.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span® par la société Sigma; les lipides polymérisés ; les lipides conjugués à de courtes chaînes d'oxyde de polyéthylène (PEG) tels que les tensioactifs non-ioniques vendus sous les dénominations commerciales Tween® par la société ICI Americas, Inc. et Triton® par la société Union Carbide Corp.; les esters de sucre tels que les mono- et di-laurate, mono- et di-palmitate, mono- et distéarate de saccharose; lesdits tensioactifs pouvant être utilisés seuls ou en mélanges.

Les phospholipides sont les lipides amphiphiles préférés.

La lécithine est le lipide amphiphile particulièrement préféré.

### • lipide solubilisant

La formulation comprend par ailleurs un lipide solubilisant comprenant au moins un glycéride d'acides gras, qui se situe dans la phase dispersée de la nanoémulsion, plus précisément dans le coeur des gouttelettes. Ce composé a pour mission principale de solubiliser le lipide amphiphile, peu soluble, dans la phase dispersée de la nanoémulsion.

Le lipide solubilisant est un lipide présentant une affinité avec le lipide amphiphile suffisante pour permettre sa solubilisation. De préférence, le lipide solubilisant est solide à température ambiante.

Dans le cas où le lipide amphiphile est un phospholipide, il peut s'agir notamment :
- d'esters d'acides gras et d'alcool gras, comme le cétylpalmitate, ou
- de dérivés du glycérol, et en particulier de glycérides obtenues par estérification de glycérol avec des acides gras.

Le lipide solubilisant utilisé est avantageusement choisi en fonction du lipide amphiphile utilisé. Il présentera généralement une structure chimique proche, afin d'assurer la solubilisation recherchée. Il peut s'agir d'une huile ou d'une cire. De préférence, le lipide solubilisant est solide à température ambiante (20°C), mais liquide à la température du corps (37°C).

Les lipides solubilisants préférés, en particulier pour les phospholipides, sont les esters d'acides gras et d'alcool gras, comme le cétylpalmitate, ou les glycérides d'acides gras, notamment d'acides gras saturés, et en particulier d'acides gras saturés comportant 8 à 18 atomes de carbone, encore préféré 12 à 18 atomes de carbone. Avantageusement, il s'agit d'un mélange de différents glycérides.

De préférence, il s'agit de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, il s'agit de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Particulièrement préférés sont les mélanges de glycérides semi-synthétiques solides à température ambiante vendus sous la dénomination commerciale Suppocire®NC par la société Gattefossé et approuvé pour l'injection chez l'homme. Les Suppocire® de type N sont obtenues par estérification directe d'acides gras et de glycérol. Il s'agit de glycérides hémi-synthétiques d'acides gras saturés de C8 à C18, dont la composition quali-quantitative est indiquée dans le tableau ci-dessous.

Les lipides solubilisants précités permettent d'obtenir une formulation sous forme de nanoémulsion avantageusement stable. Sans vouloir être lié à une théorie particulière, il est supposé que les lipides solubilisants précités permettent d'obtenir des gouttelettes dans la nanoémulsion présentant un coeur amorphe. Le coeur ainsi obtenu présente une viscosité interne élevée sans pour autant présenter de cristallinité. Or, la cristallisation est néfaste pour la stabilité de la nanoémulsion car elle conduit généralement à une agrégation des gouttelettes et/ou à une expulsion des molécules encapsulées à l'extérieur des gouttelettes. Ces propriétés physiques favorisent la stabilité physique de la nanoémulsion.

La quantité de lipide solubilisant peut varier largement en fonction de la nature et de la quantité de lipide amphiphile présent dans la phase dispersée. Généralement, le coeur des gouttelettes (comprenant le lipide solubilisant, l'éventuelle huile, l'éventuel agent d'imagerie, l'éventuel agent thérapeutique s'il est lipophile) comprend de 1 à 100% en poids, de préférence de 5 à 80% en poids et tout particulièrement de 40 à 75% en poids de lipide solubilisant.

**Composition en acides gras du Suppocire® NC de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

### • huile

La phase dispersée peut comporter par ailleurs une ou plusieurs autres huiles, qui se situe(nt) dans le coeur des gouttelettes.

Les huiles utilisées présentent de préférence une balance hydrophile-lipophile (HLB) inférieure à 8 et encore plus préférentiellement comprise entre 3 et 6. Avantageusement, les huiles sont utilisées sans modification chimique ou physique préalablement à la formation de l'émulsion.

Les huiles sont généralement choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de pépin de raisins et de tournesol ; les huiles synthétiques parmi lesquelles figurent notamment les triglycérides, di glycérides et les mono glycérides. Ces huiles peuvent être de premières expressions, raffinées ou inter-estérifiées.

Les huiles préférées sont l'huile de soja et l'huile de lin.

Généralement, si présente, l'huile sera contenue dans le coeur des gouttelettes (comprenant le lipide solubilisant, l'éventuelle huile, l'éventuel agent d'imagerie, l'éventuel agent thérapeutique s'il est lipophile) dans une proportion allant de 1 à 80% en poids, de préférence entre 5 et 50 % en poids et tout particulièrement 10 à 30% en poids.

La phase dispersée peut contenir en outre d'autres additifs tels que des colorants, stabilisants, conservateurs ou d'autres principes actifs, en quantité appropriée.

### • Co-tensioactif

La formulation comprend un co-tensioactif, qui permet de stabiliser la nanoémulsion.

Les co-tensioactifs utilisables dans les formulations utilisées dans l'invention sont généralement des tensioactifs hydrosolubles. Ils comprennent au moins une chaîne poly(oxyde d'éthylène) comprenant au moins 25, notamment au moins 30, de préférence au moins 35, unités d'oxyde d'éthylène. Le nombre d'unités d'oxyde d'éthylène est généralement inférieur à 500.

Des formulations comprenant un co-tensioactif comprenant une chaîne poly(oxyde d'éthylène) comprenant moins de 25 unités d'oxyde d'éthylène ne sont en effet pas stables. Généralement, il n'est même pas possible de préparer la nanoémulsion.

Ces nombres d'unités sont en effet particulièrement adaptés pour éviter la fuite des séquences nucléotidiques susceptibles de moduler des mécanismes endogènes d'ARN interférents hors des gouttelettes.

En effet, les inventeurs ont observé qu'une formulation ne comprenant pas un co-tensioactif n'est pas suffisamment stable.

De plus, sans vouloir être liés à une théorie particulière, la présence de la chaîne composée de motifs d'oxyde d'éthylène du co-tensioactif permettrait de protéger les séquences nucléotidiques susceptibles de moduler des mécanismes endogènes d'ARN interférents localisées à la surface des gouttelettes des protéines du milieu dans lequel la formulation est administrée/utilisée, et donc la dégradation dedites séquences nucléotidiques par ces protéines.

A titre d'exemple de co-tensioactifs, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij® (par exemple Brij® 35, 58, 78 ou 98) par la société ICI Americas Inc., les esters d'acide gras et de polyéthylèneglycol tels que les produits vendus sous les dénominations commerciales Myrj® par la société ICI Americas Inc. (par exemple Myrj® 45, 52, 53 ou 59) et les copolymères blocs d'oxyde d'éthylène et d'oxyde de propylène tels que les produits vendus sous les dénominations commerciales Pluronic® par la société BASF AG (par exemple Pluronic® F68, F127, L64, L61, 10R4, 17R2, 17R4, 25R2 ou 25R4) ou les produits vendus sous la dénomination commerciale Synperonic® par la société Unichema Chemie BV (par exemple Synperonic® PE/F68, PE/L61 ou PE/L64).

Ainsi, le co-tensioactif se situe à la fois dans la phase aqueuse continue et dans la phase dispersée. En effet, la partie hydrophobe du co-tensioactif s'insère dans les gouttelettes de la phase dispersée, alors que les chaînes polyalcoxylées sont dans la phase aqueuse continue. Dans la présente demande, les pourcentages massiques de phase dispersée décrits sont calculés en considérant que le co-tensioactif appartient à la phase dispersée.

La formulation comprend de 10% à 55% molaire de co-tensioactif par rapport à l'ensemble (lipide amphiphile / tensioactif cationique / co-tensioactif / éventuel lipide fusogène). En dessous de 10%, les formulations ne sont pas stables, et ne peuvent généralement même pas être formulées (la formation de la nanoémulsion n'est pas possible car les gouttelettes coalescent pour former deux phases). Au-delà de 55%, la complexation ultérieure de la formation « prémix » avec les séquences nucléotidiques n'a pas lieu, probablement car les charges positives du tensioactif cationique sont masquées par les chaînes poly(oxyde d'éthylène) du co-tensioactif, et donc plus accessibles pour se lier par liaison électrostatique aux séquences nucléotidiques.

Le co-tensioactif peut par ailleurs présenter d'autres effets dans l'application envisagée de la nanoémulsion.

Selon un mode de réalisation, la phase dispersée de la nanoémulsion est greffée en surface avec des molécules d'intérêts tels que des ligands biologiques, afin d'augmenter le ciblage spécifique d'un organe. Un tel greffage permet une reconnaissance spécifique de certaines cellules ou de certains organes en favorisant l'internalisation des gouttelettes de la formulation selon l'invention par les cellules cibles qui expriment le récepteur en surface. Il est ainsi possible de transfecter des cellules connues comme étant résistantes aux agents de transfection de l'art antérieur. De préférence, le greffage en surface est réalisé par couplage des molécules d'intérêts ou de leurs précurseurs avec un composé amphiphile, notamment avec le co-tensioactif. La nanoémulsion comprend alors un co-tensioactif greffé. Dans ce cas, le co-tensioactif joue le rôle d'un espaceur permettant d'accommoder les molécules d'intérêt en surface.

Les molécules d'intérêt peuvent être par exemple :
- des ligands biologiques de ciblage tels que des anticorps, peptides, saccharides, aptamères, oligonucléotides ou des composés comme l'acide folique ;
- un agent de furtivité : une entité ajoutée afin de conférer à la nanoémulsion une furtivité vis-à-vis du système immunitaire, d'augmenter son temps de circulation dans l'organisme, et de ralentir son élimination.

Par exemple, lorsque le ligand biologique est un peptide comprenant une ou plusieurs cystéine, le greffage à la chaîne oxyde d'alkylène du tensioactif peut être assuré par couplage thiol maléimide.

### • agent d'imagerie

Dans un mode de réalisation, la formulation comprend un agent d'imagerie, qui permet avantageusement de visualiser la distribution des gouttelettes dans les cellules ou le corps du patient, et donc la distribution des séquences nucléotidiques susceptibles de moduler des mécanismes endogènes d'ARN interférence.

L'agent d'imagerie peut notamment être utilisé dans les imageries de type :
- tomographie à émission de positons (Positron Emission Tomography (PET) en anglais) (l'agent d'imagerie pouvant être un composé comprenant un radionucléide, tel que ¹⁸F, ¹¹C, un chélate de cations métalliques ⁶⁸Ga, ⁶⁴Cu),
- tomographie d'émission monophotonique (TEMP) (Single photon émission computed tomography (SPECT) en anglais) (l'agent d'imagerie pouvant être un composé comprenant un radionucléide par exemple ¹²³I, ou un chélate de ^{99m}Tc ou ¹¹¹In),
- imagerie par résonance magnétique (IRM) (l'agent d'imagerie pouvant être un chélate de gadolinium ou un nanocristal magnétique tel qu'un nanocristal d'oxyde de fer, d'oxyde de manganèse ou de fer-platine FePt),
- imagerie optique ou imagerie aux rayons X (l'agent d'imagerie pouvant être un fluorophore lipophile ou un agent de contraste, par exemple une molécule iodée telle que l'iopamidol, l'amidotrizoate, ou des nanoparticules d'or).

De préférence, l'agent d'imagerie est un fluorophore lipophile permettant de réaliser de l'imagerie optique.

La nature du ou des fluorophores lipophiles utilisables n'est pas critique à partir du moment où ils sont compatibles avec l'imagerie in vivo (c'est à dire qu'ils sont biocompatibles et non toxiques). De préférence, les fluorophores utilisés comme agent d'imagerie absorbent et émettent dans le visible ou le proche infrarouge. Pour l'imagerie non invasive dans un tissu ou un organisme vivant (animal, homme) les fluorophores préférés absorbent et émettent dans le proche infrarouge. En effet, pour que la lumière d'excitation et la lumière émise par le fluorophore puissent mieux traverser les tissus, il convient d'utiliser des fluorophores absorbant et émettant dans le proche infrarouge, c'est-à-dire à une longueur d'onde comprise entre 640 et 900 nm.

A titre de fluorophore lipophile on peut par exemple citer les composés décrits dans le chapitre 13 ("Probes for Lipids and Membranes") du catalogue InVitrogen. De façon plus précise, on peut notamment citer, à titre de fluorophore, le vert d'indocyanine (ICG), les analogues d'acides gras et les phospholipides fonctionnalisés par un groupement fluorescent tels que les produits fluorescents vendus sous les dénominations commerciales Bodipy (R) tels que le Bodipy (R) 665/676 (Ex/Em.) ; les dérivés lipophiles de carbocyanines telles que le perchlorate de 1,1'--dioctadécyl-3,3,3',3'-tétraméthylindodicarbocyanine (DiD), par exemple vendu sous la référence D-307, le perchlorate de 3,3'-dihexadecyloxacarbocyanine (DiO), par exemple vendu sous la référence D1125, le perchlorate de 1,1'-dihexadecyl-3,3,3',3'-tetramethylindocarbocyanine (Dil), par exemple vendu sous la référence D384; les sondes fluorescentes dérivées de sphingolipides, de stéroïdes ou de lipopolysaccharides tels que les produits vendus sous les dénominations commerciales BODIPY ® TR céramides, BODIPY ® FL C5-lactosylcéramide, BODIPY ® FL C5-ganglioside, BODIPY ® FL cérébrosides ; les dérivés amphiphiles de cyanines, de rhodamines, de fluorescéines ou de coumarines tels que l'octadécyl rhodamine B, l'octadécyl ester de fluorescéine et la 4-heptadécyl-7-hydroxycoumarine ; et le diphénylhexatriène (DPH) et ses dérivés ; l'ensemble de ces produits étant vendus par la société Invitrogen.

Selon une forme de réalisation préférée de l'Invention, le fluorophore est le vert d'indocyanine, le perchlorate de 1,1'--dioctadécyl-3,3,3',3'-tétraméthylindodicarbocyanine, le perchlorate de 3,3'-dihexadecyloxacarbocyanine, ou le perchlorate de 1,1'-dihexadecyl-3,3,3',3'-tetramethylindocarbocyanine.

### • agent thérapeutique

La formulation selon l'invention peut comporter un agent thérapeutique.

Les agents thérapeutiques susceptibles d'être encapsulés dans la nanoémulsion selon l'invention comprennent en particulier les principes actifs agissant par voie chimique, biologique ou physique. Ainsi, il peut s'agir de principes actifs pharmaceutiques ou d'agents biologiques tels que de l'ADN, des protéines, peptides ou anticorps ou encore des agents utiles pour des thérapies physiques tels que des composés utiles pour la thermothérapie, les composés relarguant de l'oxygène singulet lorsqu'ils sont excités par une lumière utiles pour la photothérapie et des agents radioactifs. De préférence, il s'agit de principes actifs administrés de façon parentérale.

Selon son affinité lipophile ou amphiphile, l'agent thérapeutique sera encapsulé par la phase dispersée ou se situera à l'interface des deux phases.

La nature des agents thérapeutiques encapsulés dans la nanoémulsion n'est pas particulièrement limitée. Cependant, la nanoémulsion est particulièrement intéressante pour des composés peu solubles, qui sont difficiles à formuler dans les systèmes d'administration classiques et pour les principes actifs utiles pour la photothérapie, dont le rendement quantique peut être préservé.

Du fait des conditions douces du procédé de préparation, la formulation décrite est particulièrement intéressante pour l'encapsulation d'agents thérapeutiques qui se dégradent à température élevée.

Parmi les principes actifs pharmaceutiques intéressants comme agents thérapeutiques, on peut citer en particulier les agents utilisés dans le traitement du SIDA, les agents utilisés dans le traitement des maladies cardiaques, les analgésiques, les anesthésiques, les anorexigènes, les anthelmintiques, les antiallergiques, les antiangineux, les antiarythmisants, les anticholinergiques, les anticoagulants, les antidépresseurs, les antidiabétiques, les antidiurétiques, les antiémétiques, les anticonvulsivants, les antifongiques, les antihistaminiques, les antihypertenseurs, les antiinflammatoires, les anti-migraineux, les antimuscariniques, les antimycobactériens, les anticancéreux y compris les antiparkinsoniens, les antithyroïdiens, les antiviraux, les astringents, les agents bloquants, les produits sanguins, les substituts sanguins, les agents inotropes cardiaques, les agents cardiovasculaires, les agents du système nerveux central, les chélateurs, les agents de chimiothérapie, les facteurs de croissance hématopoïétiques, les corticostéroïdes, les antitussifs, les agents dermatologiques, les diurétiques, les dopaminergiques, les inhibiteurs de l'élastase, les agents endocrines, les alkaloïdes de l'ergot, les expectorants, les agents gastro-intestinaux, les agents génito-urinaires, le facteur de déclenchement de l'hormone de croissance, les hormones de croissance, les agents hématologiques, les agents hématopoïétiques, les hémostatiques, les hormones, les immunosuppresseurs, les interleukines, les analogues d'interleukines, les agents de régulation des lipides, la gonadolibérine, les myorelaxants, les antagonistes narcotiques, les nutriments, les agents nutritifs, les thérapies oncologiques, les nitrates organiques, les vagomimétiques, les prostaglandines, les antibiotiques, les agents rénaux, les agents respiratoires, les sédatifs, les hormones sexuelles, les stimulants, les sympathomimétiques, les anti-infectieux systémiques, le tacrolimus, les agents thrombolytiques, les agents thyroïdiens, les traitements pour les troubles de l'attention, les vasodilatateurs, les xanthines, les agents diminuant le cholestérol. Particulièrement visés sont les anticancéreux tels que le taxol (paclitaxel), la doxorubicine et le cisplatine.

Parmi les agents physiques ou chimiques, on peut citer notamment les isotopes radioactifs et les photo-sensibilisateurs.

Parmi les photo-sensibilisateurs, on peut citer notamment ceux appartenant à la classe des tétrapyrroles comme les porphyrines, les bactériochlorines, les phtalocyanines, les chlorines, les purpurines, les porphycènes, les phéophorbides, ou encore ceux appartenant à la classe des texaphyrines ou des hypericines. Parmi les photo-sensibilisateurs de première génération, on peut mentionner l'hémato-porphyrine et un mélange de dérivés d'hémato-porphyrine (HpD) (vendu sous la marque commerciale Photofrin® par Axcan Pharma). Parmi les photo-sensibilisateurs de seconde génération, on peut mentionner le méta-tetra-hydroxyphenyl chlorine (mTHPC ; nom commercial Foscan®, Biolitec AG) et le dérivé monoacide du cycle A de la benzoporphyrine (BPD-MA vendu sous la marque commerciale Visudyne® par QLT et Novartis Opthalmics). Les formulations des photo-sensibilisateurs de seconde génération qui associent à ces photo-sensibilisateurs une molécule (lipide, peptide, sucre etc..) qualifiée de transporteur qui permet leur acheminement sélectif au niveau du tissu tumoral sont appelées photo-sensibilisateurs de troisième génération.

Parmi les agents biologiques, on peut mentionner les oligonucléotides, de l'ADN, de l'ARN, des siARN, les peptides et les protéines et les saccharides.

Bien entendu, l'agent thérapeutique peut être formulé directement sous sa forme active ou sous forme d'un prodrug. Par ailleurs, il est envisagé que plusieurs agents thérapeutiques puissent être formulés en association dans la nanoémulsion.

La quantité d'agent thérapeutique dépend de l'application visée ainsi que de la nature de l'agent. Toutefois, on cherchera généralement à formuler la nanoémulsion avec une concentration maximale en agent thérapeutique, notamment lorsqu'il s'agit d'agents thérapeutiques peu solubles, afin de limiter le volume et/ou la durée d'administration au patient.

Or il a été constaté que la présence du lipide solubilisant dans la phase dispersée permet d'incorporer une quantité importante de composés mêmes hydrophobes ou amphiphiles.

### • Proportion de coeur dans les gouttelettes

Généralement, la proportion molaire des composants du coeur des gouttelettes par rapport aux composants des gouttelettes est de 10 à 80%, notamment de 25 à 75%, de préférence de 33,35 à 73,99%. Autrement dit, la proportion molaire (mol/mol) de l'ensemble (lipide solubilisant / éventuelle huile / éventuel agent d'imagerie / éventuel agent thérapeutique lipophile) par rapport à la phase dispersée (c'est-à-dire à tous les composants de la phase dispersée) est généralement de 10 à 80%, notamment de 25 à 75%, de préférence de 33,35 à 73,99%.

Généralement, la proportion massique (wt/wt) des composants du coeur des gouttelettes par rapport aux composants des gouttelettes est de 10 à 60%, notamment de 20 à 60%, de préférence de 23,53 à 59,51%. Autrement dit, la proportion massique de l'ensemble (lipide solubilisant / éventuelle huile / éventuel agent d'imagerie / éventuel agent thérapeutique lipophile) par rapport à la phase dispersée (c'est-à-dire à tous les composants de la phase dispersée) est généralement de 10 à 60%, notamment de 20 à 60%, de préférence de 23,53 à 59,51%.

Ces proportions molaires et/ou massiques sont particulièrement adaptées pour que la formulation « premix » soit stable au stockage, notamment qu'elle soit stable en étant stockée plus de 28 jours à 40°C, voire plus de 300 jours à 40°C. La stabilité peut notamment être mesurée en suivant la taille des gouttelettes, leur index de polydispersité et/ou leur potentiel zéta (par exemple par diffusion quasi-élastique de la lumière par un appareil de type ZetaSizer, Malvern). Cette stabilité de la formulation « premix » est importante pour les applications envisagées. En effet, comme détaillé ci-après, la formulation « premix » est utilisée pour préparer la formulation « finale » comprenant une séquence nucléotidique susceptible de moduler des mécanismes endogènes d'interférence par ARN. Il est particulièrement intéressant de pouvoir stocker la formulation « premix » et d'effectuer la complexation avec ladite séquence juste avant utilisation de la formulation « finale ».

### • Phase aqueuse

La phase aqueuse de la nanoémulsion utilisée dans l'invention est de préférence constituée d'eau et/ou d'un tampon tel qu'un tampon phosphate comme par exemple du PBS ("Phosphate Buffer Saline") ou d'une solution saline, notamment de chlorure de sodium.

Selon un mode de réalisation, la phase aqueuse continue comporte également un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

La proportion de phase dispersée et de phase aqueuse est très variable. Cependant, le plus souvent, les nanoémulsions seront préparées avec 1 à 50%, de préférence 5 à 40% et tout particulièrement 10 à 30% en poids de phase dispersée et 50 à 99%, de préférence 60 à 95% et tout particulièrement 70 à 90 % en poids de phase aqueuse.

### • Nanoémulsion sous forme de gel

Dans un mode de réalisation, la viscosité de la formulation est supérieure à 1 poise (0,1 Pa.s) à 25°C.

Dans ce mode de réalisation, la nanoémulsion utilisée se présente sous forme de « gel ». On entend par le terme « gel » habituellement un système biphasique solide-liquide thermodynamiquement stable, constitué d'un double réseau interpénétré continu tridimensionnel, l'un solide et le second liquide. Un tel gel est un système biphasique liquide-solide dont le réseau solide retient une phase liquide. Bien que les gels puissent être considérés comme solides, ils présentent des propriétés propres aux solides (rigidité structurelle, élasticité à la déformation) comme aux liquides (pression de vapeur, de compressibilité et conductivité électrique).

Dans le cas d'une nanoémulsion sous forme de gel, le réseau tridimensionnel est formé par les gouttelettes, les interstices entre gouttelettes étant remplis de phase continue. Les liaisons entre les unités du réseau, à savoir les gouttelettes, reposent principalement sur des interactions électrostatiques (paires d'ions). Ces interactions électrostatiques existent principalement entre les séquences nucléotidiques susceptibles de moduler des mécanismes endogènes d'ARN interférence et les tensioactifs cationiques de gouttelettes adjacentes.

Une nanoémulsion sous forme de gel montre donc une résistance à la pression et est capable de maintenir une forme définie, ce qui peut être avantageux selon la forme et/ou la voie d'administration souhaitée.

Pour mettre en évidence que la nanoémulsion est sous forme de gel, on peut réaliser des études rhéologiques permettant d'évaluer les propriétés viscoélastiques, et/ou des études plus structurelles montrant les liaisons entre les gouttelettes formant le réseau tridimensionnel (diffraction aux rayons X, neutrons...). En effet, une nanoémulsion sous forme de gel possède une viscosité et un coefficient d'élasticité plus important qu'une nanoémulsion liquide. La nanoémulsion sous forme de gel peut, en fonction de la concentration de gouttelettes et donc de la fraction massique en phase dispersée, se trouver à l'état de liquide visqueux, de solide viscoélastique ou de solide élastique. Par rapport à la phase dispersante aqueuse, dont la viscosité est proche de celle de l'eau (1 mPa.s à 25°C), la nanoémulsion est considérée comme un liquide visqueux lorsque sa viscosité est 10 fois plus élevée que celle de l'eau, soit > 10 mPa.s à 25°C. Par ailleurs, lorsque l'on procède à la mesure rhéologique des modules de G' (module de conservation en cisaillement) et G" (module de perte en cisaillement), on considère que la nanoémulsion est sous forme d'un liquide visqueux lorsque G" >G'. Lorsque G' devient proche de G", la nanoémulsion est à l'état de solide viscoélastique. Lorsque G" < G', on est à l'état de solide élastique. Dans ce mode de réalisation, la nanoémulsion se présente de préférence à l'état liquide visqueux ou de solide viscoélastique, car la viscosité est suffisamment modérée dans ces états pour permettre des applications impliquant une administration par injection. La viscosité et le coefficient d'élasticité peuvent être mesurés par un rhéomètre cône-plan ou par un rhéomètre Couette. La viscosité d'une nanoémulsion liquide est généralement inférieure à 1 poise, voire même souvent inférieure à 0,01 poise. La nanoémulsion utilisée dans ce mode de réalisation de l'invention a généralement une viscosité supérieure à 1 poise, et pourra avoir une viscosité allant jusqu'à celle d'un solide (plus de 1000 poises). La nanoémulsion de la présente invention a généralement une viscosité de 1 à 1000 poises, préférentiellement de 1 à 500 poises et encore plus préférentiellement entre 1 et 200, ces valeurs étant données à 25°C. Une viscosité supérieure à 1 poise est en effet adaptée pour que les gouttelettes de la phase dispersée forment un réseau tridimensionnel à l'intérieur de la phase continue. En effet, il a été constaté que en dessous de 1 poise, les gouttelettes ne sont généralement pas assez proches les unes des autres,. Au dessus de 1000 poises, on obtient un système quasi-solide. La nanoémulsion est alors trop visqueuse ce qui rend son utilisation difficile. De même, alors que le coefficient d'élasticité est généralement inférieur à 10 dans le cas d'une nanoémulsion liquide, le coefficient d'élasticité d'une nanoémulsion sous forme de gel est généralement supérieur à 10. Les études structurelles, notamment les diffractions aux rayons X ou aux neutrons, permettent également de différencier l'organisation d'une nanoémulsion liquide, de l'organisation d'une nanoémulsion sous forme de gel. En effet, les pics du diffractogramme obtenu pour une nanoémulsion liquide sont caractéristiques de la structure des gouttelettes de phase dispersée (grand angles de diffraction caractéristiques de distances courtes), alors que les pics du diffractogramme d'une nanoémulsion sous forme de gel sont caractéristiques non seulement de la structure des gouttelettes (grand angles de diffraction caractéristiques de distances courtes) mais aussi de l'organisation de ces gouttelettes en réseau tridimensionnel (faibles angles de diffraction caractéristiques de distances plus grandes).

La nanoémulsion utilisée dans ce mode de réalisation de l'invention est avantageusement sous forme de gel dispersible, c'est-à-dire que les gouttelettes formant le réseau tridimensionnel peuvent être relarguées dans la phase continue sous certaines conditions par « dégélification » du système gel, également dénommée « désagrégation » dans la présente demande. La désagrégation est observée par ajout de phase continue au gel, par mise en contact avec les fluides physiologiques lors de l'administration de la nanoémulsion ou par augmentation de la température. En effet, ajouter de la phase continue entraîne une différence de pression osmotique entre l'intérieur du gel et la phase continue. Le système tendra donc à diminuer, jusqu'à annuler, cette différence de pression osmotique en libérant les gouttelettes dans l'excès de phase continue, jusqu'à obtenir une concentration en gouttelettes homogène dans l'ensemble du volume de phase continue. De même augmenter suffisamment la température du système revient à donner aux différentes gouttelettes une énergie thermique supérieure aux énergies mises en jeu dans les liaisons, par exemple les liaisons hydrogène, et ainsi à rompre ces liaisons et libérer les gouttelettes du réseau tridimensionnel. Ces températures dépendent de la composition du gel et plus particulièrement de la taille des gouttelettes et de la longueur des chaînes polyalcoxylées du co-tensioactif. La désagrégation de la nanoémulsion sous forme de gel peut être suivie par diffraction aux rayons X, par calorimétrie différentielle à balayage (DSC) ou par résonance magnétique nucléaire (RMN). En suivant par diffraction aux rayons X la désagrégation de la nanoémulsion sous forme de gel, on observe une évolution du spectrogramme, c'est-à-dire une diminution de l'intensité des angles faibles (caractéristiques de l'organisation des gouttelettes en réseau tridimensionnel) (comme décrit dans Matija Tomsic, Florian Prossnigg, Otto Glatter 'Journal of Colloid and Interface Science' Volume 322, Issue 1, 1 June 2008, Pages 41-50). La désagrégation peut également être suivie par DSC. Un pic apparaît sur le thermogramme lors de la transition nanoémulsion sous forme de gel / nanoémulsion liquide en montée en température. Enfin, une étude RMN peut aussi permettre de suivre la désagrégation par mesure du coefficient de diffusion associé à chaque gouttelette en distinguant une nanoémulsion liquide d'une nanoémulsion sous forme de gel. En effet, le coefficient de diffusion est très significativement diminué dans le cas d'une nanoémulsion sous forme de gel (il est alors généralement inférieur à 0.01µm²/s), où le système est figé. (WESTRIN B. A.; AXELSSON A.; ZACCHI G. 'Diffusion measurement in gels', Journal of controlled release 1994, vol. 30, n°3, pp. 189-199).

### • Emulsion dans laquelle les gouttelettes sont liées entre-elles de façon covalente

Dans un mode de réalisation, la formulation comprend un tensioactif de formule (I) suivante :

(L₁-X₁-H₁-Y₁)ᵥ-G-(Y₂-H₂-X₂-L₂)_{w} (I),

dans laquelle :
- L₁ et L₂ représentent indépendamment des groupes lipophiles,
- X₁, X₂, Y₁, Y₂ et G représentent indépendamment un groupe de liaison,
- H₁ et H₂ représentent indépendamment des groupes hydrophiles comprenant une chaîne polyalcoxylée,
- v et w sont indépendamment un nombre entier de 1 à 8,
et les gouttelettes de la phase dispersée sont liées entre-elles de façon covalente par le tensioactif de formule (I).

Le tensioactif de formule (I) se situe partiellement dans la phase aqueuse continue et partiellement dans la phase dispersée. En effet, le tensioactif de formule (I) comprend deux groupes lipophiles (L₁ et L₂) et deux groupes hydrophiles (H₁ et H₂). Les groupes hydrophiles sont situés majoritairement à la surface des gouttelettes, dans la phase aqueuse continue alors que les groupes lipophiles se situent dans les gouttelettes de la formulation.

Plus précisément, le groupe lipophile L₁ se situe dans certaines gouttelettes, et le groupe L₂ dans des gouttelettes adjacentes. Les gouttelettes sont donc reliées de façon covalente entre-elles par le groupe -(X₁-H₁-Y₁)ᵥ-G-(Y₂-H₂-X₂)_{w}- du tensioactif de formule (I).

Les groupes X₁ et X₂ sont des groupes de liaison liant les groupes lipophiles et hydrophiles. Le groupe G est un groupe de liaison entre les deux parties [lipophile-hydrophile] du tensioactif de formule (I).Les groupes Y₁ et Y₂ sont des groupes de liaison liant le groupe G à ces deux parties [lipophile-hydrophile].

La formulation de ce mode de réalisation peut avantageusement être mise en forme (par exemple en la plaçant dans un moule ou un récipient ayant une forme donnée), et rester dans la forme désirée selon l'application souhaitée. Ce mode de réalisation de l'invention est donc particulièrement adapté lorsque la formulation est utilisée sous forme de gélule, de gel, d'ovule ou de patch.

D'autre part, elle résiste à la dilution à une phase aqueuse. Plus précisément, lorsqu'une phase aqueuse est ajoutée à cette formulation, celle-ci reste en forme et ne se dilue pas. Dans le milieu, on observe d'une part la formulation comprenant les gouttelettes, et d'autre part une phase aqueuse essentiellement exempte de gouttelettes.

Sans vouloir être liés à une théorie particulière, il semble que ces propriétés de cette formulation puissent être expliquées par la présence des liaisons covalentes entre les gouttelettes, qui confèrent une cohésion très forte entre elles.

Dans un mode de réalisation, dans la formule (I) susmentionnée :
- L₁ et L₂ sont indépendamment choisis parmi :
   - un groupe R ou R-(C=O)-, où R représente une chaîne hydrocarbonée linéaire comprenant de 11 à 23 atomes de carbone,
   - un ester ou un amide d'acides gras comprenant de 12 à 24 atomes de carbone et de phosphatidyléthanolamine, tel que le distéaryl phosphatidyléthanolamine (DSPE), et
   - un poly(oxyde de propylène), et/ou
- X₁, X₂, Y₁ et Y₂ sont indépendamment choisis parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents, et/ou
- H₁ et H₂ sont indépendamment choisis parmi un poly(oxyde d'éthylène) comprenant typiquement de 3 à 500 unités oxyde d'éthylène, de préférence de 20 à 200 unités oxyde d'éthylène, et/ou
- G comprend au moins un groupe G' ayant l'une des formules suivantes (les groupes Y₁ et Y₂ étant reliés à gauche et à droite des formules décrites ci-dessous): 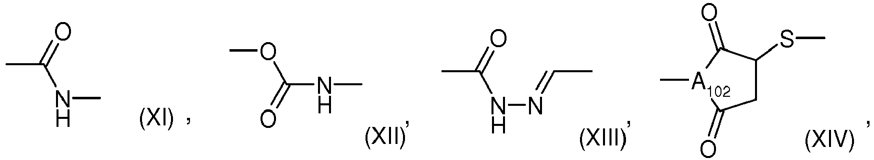 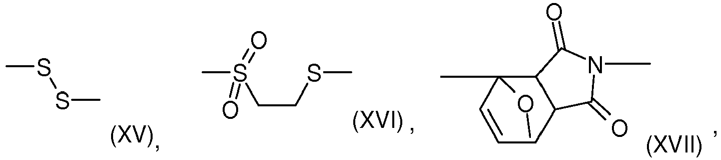 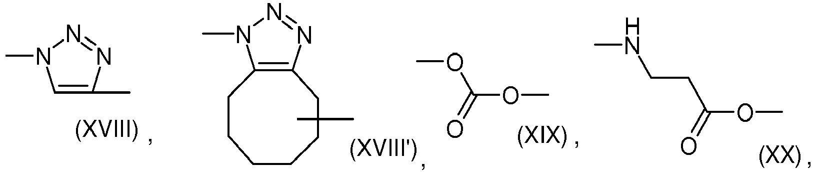 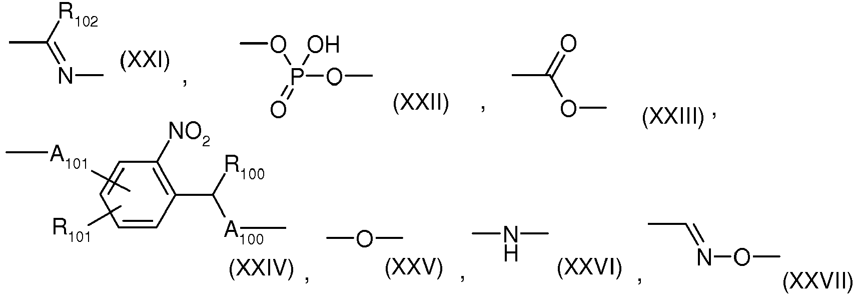 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.
   Par la formule 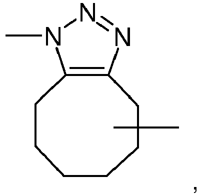 on entend que le groupe Y₂ peut être relié à n'importe lequel des six atomes du cyclooctyle et par la formule 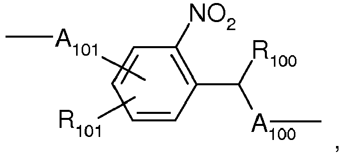 on entend que les groupe A₁₀₁ et R₁₀₁ peuvent être relié à n'importe lequel des quatre atomes du phényle.

Notamment, v et w représentent indépendamment 1 ou 2. v et w représentent de préférence 1.

Le groupe G peut comprendre un ou plusieurs des groupes G' définis ci-dessus.

Ainsi, dans un premier mode de réalisation, le groupe G est constitué d'un groupe G'. Dans ce mode de réalisation, dans la formule (I), v et w représentent 1.

Dans un second mode de réalisation, le groupe G répond à la formule -G'-Y₃-G'-dans laquelle :
- Y₃ représente un groupe de liaison, notamment choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents
- chacun des G' représente indépendamment un groupe de formule (XI) à (XXVI) susmentionnés, et de préférence, les deux groupes G' de la formule -G'-Y₃-G'- sont identiques.
Dans ce mode de réalisation, dans la formule (I), v et w représentent 1.
Ce mode de réalisation est particulièrement intéressant lorsque les deux groupes G' sont identiques et comprennent une fonction clivable. En effet, il suffit alors de cliver une seule des deux fonctions pour rompre les liaisons covalentes entre les gouttelettes de la formulation.

Dans un troisième mode de réalisation, le groupe G est un dendrimère comprenant (v+w) groupes G'. Le groupe G peut notamment être un dendrimère comprenant plusieurs groupes G', tel qu'un dendrimère comprenant un groupe polyamidoamine (PAMAM). Par exemple, le groupe G peut avoir l'une des formules (XXX) à (XXXIII) suivantes, qui comprennent :
- 4 groupes G' de formule (XXVI). v et w représentent 2.
- 4 groupes G' de formule (XXIV) où R₁₀₁ représente -O-Me, A₁₀₁ représente -NH-, R₁₀₀ représente un méthyle et A₁₀₀ représente -NH-. v et w représentent 2.
- 4 groupes G' de formule (XIV). v et w représentent 2.
- 16 groupes G' de formule (XXVI). v et w représentent 8.

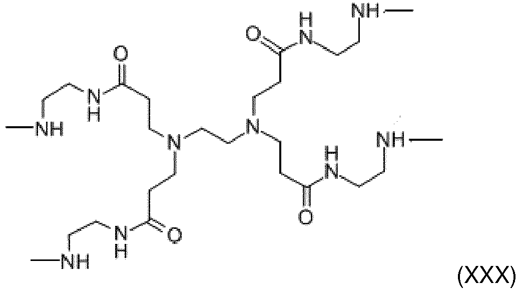

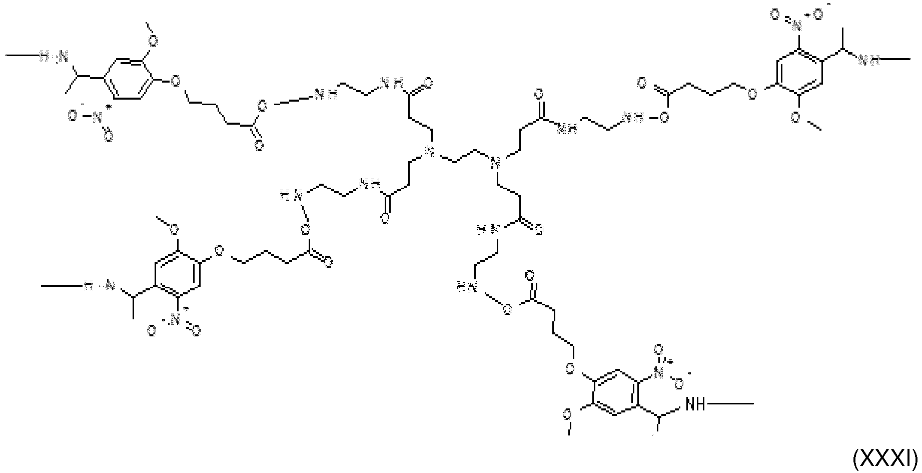

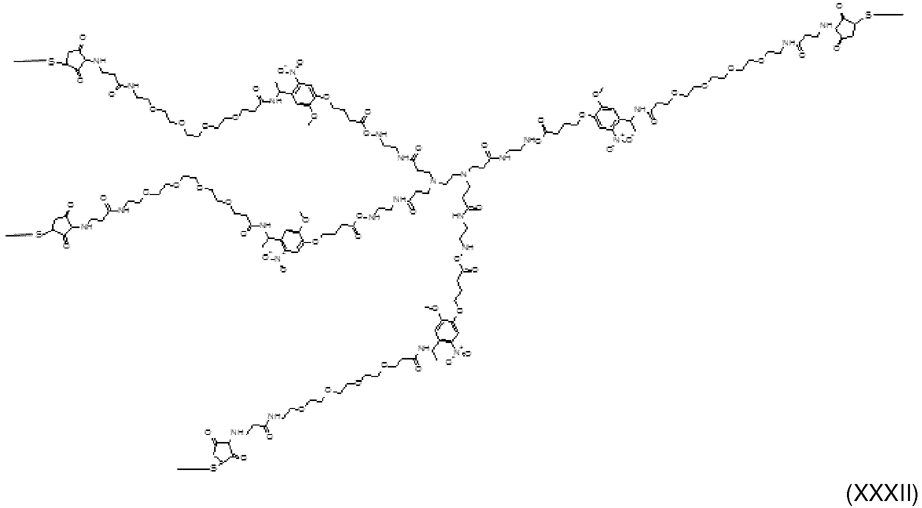

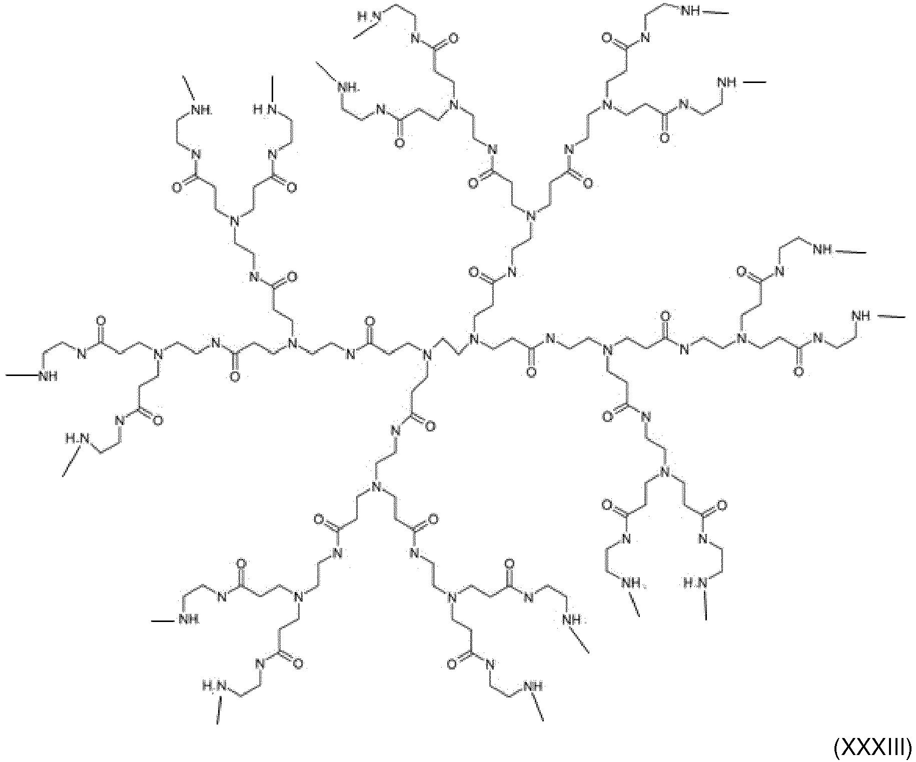

Lorsque L₁ et/ou L₂ représentent un groupe R-(C=O)-, où R représente une chaîne hydrocarbonée linéaire comprenant de 11 à 23 atomes de carbone, L₁ et/ou L₂ représentent des groupes issus d'acide gras comprenant de 12 à 24 atomes de carbone.

Par «L₁ et L₂ représentent un ester ou un amide d'acides gras comprenant de 12 à 24 atomes de carbone et de phosphatidyléthanolamine», on entend qu'ils représentent un groupe de formule : 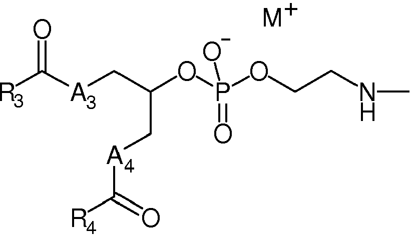 dans laquelle
- R₃ et R₄ représentent indépendamment une chaîne hydrocarbonée linéaire comprenant de 11 à 23 atomes de carbone,
- A₃ et A₄ représentent O ou NH, et
- M représente H ou un cation.

De préférence, L₁ et L₂ sont identiques et/ou X₁ et X₂ sont identiques et/ou H₁ et H₂ sont identiques. Des tensioactifs de formule (I) particulièrement préférés sont ceux dans lesquels L₁ et L₂ sont identiques, X₁ et X₂ sont identiques et.H₁ et H₂ sont identiques. Ces tensioactifs sont en effet des composés symétriques et sont donc généralement plus faciles à synthétiser, et donc moins coûteux.

Dans un mode de réalisation, dans la formule (I) susmentionnée, les radicaux L₁-X₁-H₁- et/ou L₂-X₂-H₂- consistent en l'un des groupes de formules suivantes (le groupe Y₁ ou Y₂ étant relié à droite des formules décrites ci-dessous): 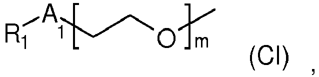 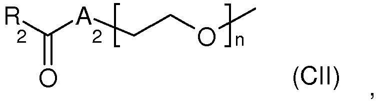 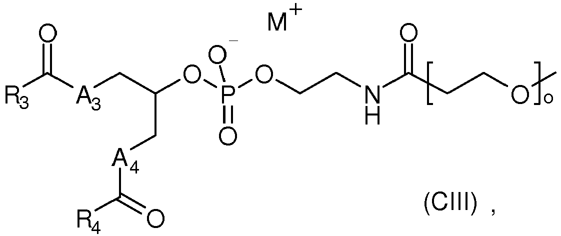 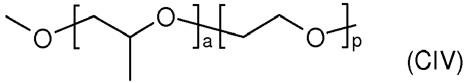 dans lesquelles :
- R₁, R₂, R₃ et R₄ représentent indépendamment une chaîne hydrocarbonée linéaire comprenant de 11 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation.

Les radicaux L₁-X₁-H₁-et/ou L₂-X₂-H₂- de formule (CII) sont préférés. En effet, ils sont faciles à préparer (notamment par formation d'un ester ou d'une amide entre un acide gras et un dérivé de poly(éthylène glycol). De plus, une formulation comprenant un tensioactif comprenant un radical L₁-X₁-H₁-et/ou L₂-X₂-H₂- de formule (CII) peut généralement être préparé avec une quantité plus importante de ce tensioactif qu'une formulation comprenant un tensioactif comprenant un radical L₁-X₁-H₁-et/ou L₂-X₂-H₂- de formule (CIII). Or, plus la proportion de tensioactif de formule (I) dans la formulation est élevée, plus la cohésion entre les gouttelettes est grande, et plus la formulation reste en forme et résiste à la dilution. Ainsi, ces deux propriétés peuvent être encore exacerbées pour une formulation comprenant un tensioactif comprenant un radical L₁-X₁-H₁-et/ou L₂-X₂-H₂- de formule (CII).

Les radicaux L₁-X₁-H₁-et/ou L₂-X₂-H₂- de formule (CII) avec A₂ représente NH sont particulièrement préférés, car les tensioactifs comprenant de tels radicaux permettent d'éviter la fuite des agents d'intérêts lipophiles éventuellement présents hors des gouttelettes de la formulation plus efficacement que des tensioactifs comprenant des radicaux L₁-X₁-H₁-et/ou L₂-X₂-H₂- de formule (CII) avec A₂ représente O.

Dans un mode de réalisation, dans la formule (I), v et w représentent 1, L₁ et L₂ sont indépendamment R-(C=O)-, où R représente une chaîne hydrocarbonée linéaire comprenant de 11 à 23 atomes de carbone, H₁ et H₂ sont indépendamment des poly(oxyde d'éthylène) comprenant de 3 à 500 unités oxyde d'éthylène, X₁ et X₂ représentent -O- ou -NH-, G est constitué d'un groupe G' représentant -S-S- (groupe de formule (XV) ci-dessus) et Y₁ et Y₂ représentent -CH₂-CH₂-NH-CO-CH₂-CH₂- (groupe Alk-Z-Alk ci-dessus avec Alk représente -CH₂-CH₂- et Z représente -NH-(CO)-) et le tensioactif de la formulation a alors la formule (I') suivante : 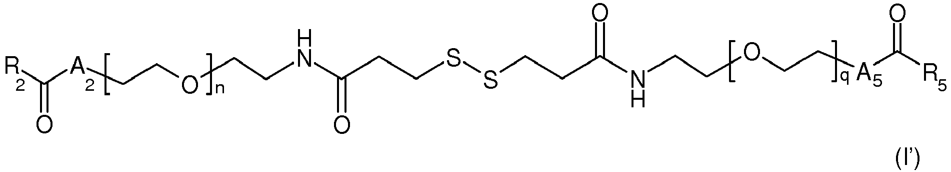 dans laquelle :
- R₂ et R₅ représentent indépendamment une chaîne hydrocarbonée linéaire comprenant de 11 à 23 atomes de carbone, de préférence 17,
- A₂ et A₅ représentent O ou NH, de préférence NH, et
- n et q représentent indépendamment des nombres entiers de 3 à 500, de préférence de 20 à 200.

Dans un mode de réalisation, les groupes H₁ et H₂ sont indépendamment choisis parmi un poly(oxyde d'éthylène) comprenant plus de 3 unités de poly(oxyde d'éthylène), voire plus de 20 unités, notamment plus de 50 (dans les formules susmentionnées, m, n, o, p et/ou q sont de préférence supérieurs à 3, voire 20, notamment plus de 50).

Dans un mode de réalisation, le groupe G du tensioactif de formule (I) de la formulation comprend une fonction clivable, notamment à certains pH (pH basique ou acide), par des enzymes, par la lumière (lumière visible, ultraviolets ou infrarouge) et/ou au-delà de certaines températures. Généralement, le groupe G comprend alors un groupe G' comprenant une fonction clivable.

Par exemple :
- la fonction β-cétoaminoester du groupe G de formule (XX) est clivable à pH acide (typiquement autour de 5),
- la fonction disulfure du groupe G de formule (XV) est clivable aux ultraviolets ou par des enzymes telles que des thioréductases,
- la fonction amide du groupe G de formule (XI) est clivable par des enzymes telles que des protéases,
- la fonction phosphate du groupe G de formule (XXII) est clivable par des enzymes telles que des phosphatases,
- la fonction imine des groupes G de formules (XXI) et (XIII) sont clivables à pH acide ou au-delà de certaines températures,
- le cycle cyclohexène du groupe G de formule (XVII) est clivable au-delà de certaines températures (par rétro Diels-Alder),
- la fonction carbonate du groupe G de formule (XIX) et la fonction carbamate du groupe G de formule (XII) sont clivables à pH acide.

L'homme du métier, au vu de ses connaissances générales, sait quelles fonctions sont clivables et dans quelles conditions. Il est notamment à même de choisir la fonction du groupe G' du tensioactif de formule (I) pour qu'elle soit clivable dans les conditions rencontrées lors de l'administration de la formulation selon l'invention.

De préférence, le rapport de la masse de tensioactif de formule (I) sur la masse de l'ensemble (tensioactif de formule (I) / co-tensioactif) est supérieur ou égal à 15%. Il a en effet été observé que de telles formulations sont plus faciles à préparer.

### • Taille des gouttelettes de la formulation « premix »

Les gouttelettes de la formulation « premix » définie ci-dessus ont généralement un diamètre compris entre 20 et 200 nm. Ce diamètre peut notamment être mesuré par diffusion quasi-élastique de la lumière sur un appareil ZetaSizer, Malvern.

Il est possible d'obtenir des gouttelettes de taille plus spécifique en adaptant les pourcentages des composants de la nanoémulsion.

Pour une formulation comprenant des gouttelettes de taille comprise entre 20 et 40 nm, on préférera utiliser une formulation comprenant au moins 5% molaire de lipide amphiphile et :
- de 25 à 45% molaire de co-tensioactif (en dessous de 25% molaire, la formulation peut présenter des problèmes de stabilité), et/ou
- de 15 à 50% molaire de tensioactif cationique.

Pour une formulation comprenant des gouttelettes de taille comprise entre 40 et 100 nm, on préférera utiliser une formulation comprenant au moins 5% molaire de lipide amphiphile et :
- de 45 à 50% molaire de co-tensioactif (en dessous de 45% molaire, la formulation peut présenter des problèmes de stabilité. Au-delà de 50%, l'efficacité en transfection de la formulation « finale » après complexation avec les séquences nucléotidiques est moindre), et/ou
- de 30 à 40% molaire de tensioactif cationique. (en dessous de 30% molaire, l'efficacité en transfection de la formulation « finale » après complexation avec les séquences nucléotidiques est moindre. Au-delà de 40%, la formulation peut présenter des problèmes de stabilité).

Pour une formulation comprenant des gouttelettes de taille comprise entre 130 et 175 nm, on préférera utiliser une formulation comprenant au moins 5% molaire de lipide amphiphile et de 15 à 70% molaire d'au moins un tensioactif cationique et :
- de 10 à 25% molaire, notamment de 10 à 15% de co-tensioactif.

Les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

### [Formulation comprenant une séquence nucléotidique susceptible de moduler des mécanismes endogènes d'ARN interférents] (dite aussi « formulation « finale »)

### • séquence nucléotidique susceptible de moduler des mécanismes endogènes d'ARN interférents

En complexant la formulation « premix » définie ci-dessus avec une séquence nucléotidique susceptible de moduler des mécanismes endogènes d'interférence par ARN, on obtient la formulation « finale » utile pour la délivrance desdites séquences.

Ainsi, la formulation peut en outre comprendre une séquence nucléotidique mono ou double brin, comprenant moins de 200 bases pour une séquence nucléotidique mono brin ou moins de 200 paires de base pour une séquence nucléotidique double brin, et susceptible de moduler des mécanismes endogènes d'ARN (acide ribonucléique) interférents, telle que :
- un petit ARN interférent (siARN) (« short-interfering RNA » ou « small interfering RNA » siRNA en anglais)
- un acide nucléique bloqué (« Locked Nucleic Acid » LNA en anglais) :
- un microARN synthétique, dit Mimic (« MicroRNA » ou « miRNA » en anglais).

Dans un mode de réalisation, ladite séquence nucléotidique est un siARN.

Un siARN est une séquence nucléotidique d'ARN double brin. C'est une séquence nucléotidique naturelle ou synthétique.

Un siARN est susceptible de cibler un transcrit d'intérêt, c'est à dire que la séquence nucléotidique d'un des brins du siARN est complémentaire de la séquence nucléotidique du transcrit d'intérêt. La taille de chaque brin du double brin du siARN varie généralement de 15 à 30 nucléotides, de préférence de 19 à 25 nucléotides, en particulier de 19 à 21 nucléotides. Deux deoxythymidine sont généralement ajoutés en partie 3' de chacun de ses brins pour en augmenter la stabilité. Ainsi, un siARN dont des deoxythymidine ont été greffés à ses parties 3' ne sort pas de la définition d'un siARN selon la présente demande. Un siARN permet de diminuer l'expression d'une protéine cible par interférence avec l'ARN messager codant pour cette protéine.

Dans un mode de réalisation, ladite séquence nucléotidique est un acide nucléique bloqué.

Un acide nucléique bloqué est une séquence nucléotidique d'ARN et/ou d'ADN mono brin dont au moins un des acides nucléiques contient un pont méthylène entre l'hydroxyle en position 2 et l'atome de carbone 4 du ribose. C'est une séquence nucléotidique synthétique.

Un acide nucléique bloqué est un inhibiteur de microARN et permet de réguler l'expression d'une ou plusieurs protéines cibles dont les ARNm étaient en interférence avec les séquences ARN issues du dit microARN. La régulation est le plus souvent une levée d'inhibition d'expression des protéines.

Dans un mode de réalisation, ladite séquence nucléotidique est un microARN.

Un microARN est une séquence nucléotidique d'ARN simple brin (de l'ordre de la centaine de bases). C'est une séquence nucléotidique synthétique.

Un microARN permet de réguler l'expression d'une ou plusieurs protéines cibles par interférence avec un ou plusieurs ARNm codant respectivement pour ces protéines.. La régulation est le plus souvent une inhibition de l'expression des protéines.

Lesdites séquences nucléotidiques sont maintenues à la surface des gouttelettes de la phase dispersée de la formulation grâce aux interactions électrostatiques avec le tensioactif cationique. Elles sont donc localisées à la surface des gouttelettes, au niveau de la couronne des gouttelettes, du côté hydrophile de la couronne.

Outre l'éventuelle liaison des siARN à des deoxythymidine mentionnées ci-dessus, lesdites séquences nucléotidiques ne sont pas modifiées chimiquement et elles ne sont pas dénaturées. En particulier, lesdites séquences nucléotidiques ne sont pas liées de façon covalente aux autres composants des gouttelettes. Notamment, lesdites séquences nucléotidiques ne sont liées de façon covalente ni au co-tensioactif, ni au lipide amphiphile, ni à l'éventuel agent d'imagerie. En effet, lesdites séquences nucléotidiques sont uniquement liées aux gouttelettes de la nanoémulsion par interactions électrostatiques avec les tensioactifs cationiques. Ceci est très avantageux car lesdites séquences nucléotidiques, une fois libérées dans leur site d'action, ne sont pas dénaturées et peuvent jouer le rôle attendu. De plus, il n'est pas nécessaire de préparer des dérivés de séquences nucléotidiques, ce qui est coûteux. Les séquences nucléotidiques disponibles dans le commerce peuvent donc être complexées aux gouttelettes sans modification préalable.

Les gouttelettes de la formulation « finale » ont généralement un diamètre compris entre 20 et 250 nm, typiquement entre 40 et 200 nm. Ce diamètre peut notamment être mesuré par diffusion quasi-élastique de la lumière sur un appareil ZetaSizer, Malvern.

### • Localisation des composants des gouttelettes

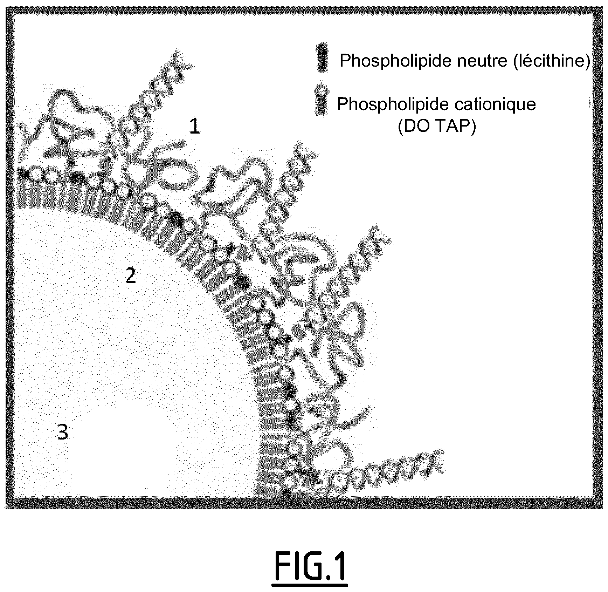

Comme illustré sur la figure 1, les gouttelettes de la formulation selon l'invention s'organisent sous forme de coeur - couronne, où :
- le coeur comprend :
   - le lipide solubilisant,
   - l'éventuelle huile,
   - l'éventuel agent d'imagerie,
   - l'éventuel agent thérapeutique s'il est lipophile,
- la couronne comprend :
   - le lipide amphiphile,
   - le tensioactif cationique,
   - le co-tensioactif (éventuellement greffé avec une molécule d'intérêt),
   - la séquence nucléotidique susceptible de moduler des mécanismes endogènes d'ARN interférence,
   - l'éventuel lipide fusogène,
   - l'éventuel agent thérapeutique s'il est amphiphile,
   - l'éventuel tensioactif de formule (I).

Cette organisation est la même dans la formulation « premix », excepté que dans ce cas, la couronne est exempte de séquence nucléotidique susceptible de moduler des mécanismes endogènes d'interférence par ARN.

### [Kit]

Selon un deuxième aspect, l'invention concerne un kit comprenant la formulation « prémix » telle que définie ci-dessus, et séparément, au moins une séquence nucléotidique mono ou double brin, comprenant moins de 200 bases pour une séquence nucléotidique mono brin ou moins de 200 paires de base pour une séquence nucléotidique double brin, et susceptible de moduler des mécanismes endogènes d'ARN (acide ribonucléique) interférence.

Dans le kit, la formulation « prémix » est séparée physiquement d'au moins une séquence nucléotidique. On peut par exemple utiliser un kit comprenant au moins deux récipients, un pour la formulation « prémix », et au moins un pour une séquence nucléotidique, de préférence plusieurs contenant chacun des séquences nucléotidiques de nature différente.

Comme explicité ci-dessus, la formulation « premix », notamment grâce à :
- la proportion molaire de tensioactif cationique dans la couronne des gouttelettes,
- la proportion molaire de lipide amphiphile dans la couronne des gouttelettes,
- la proportion molaire de co-tensioactif dans la couronne des gouttelettes,
- le fait que le co-tensioactif comprenne une chaîne poly(oxyde d'éthylène) comprenant au moins 25 unités d'oxyde d'éthylène,
- la présence de lipide solubilisant dans le coeur des gouttelettes, et/ou
- la proportion molaire et/ou massique de coeur dans les gouttelettes,
est avantageusement stable. Cette stabilité de la formulation « premix » permet de stocker à long terme la formulation « premix », ce qui est un prérequis pour pouvoir stocker et commercialiser le kit défini ci-dessus.

Il est ainsi avantageusement possible de préparer la formulation « finale » à partir de la formulation « premix » et de la séquence juste avant utilisation de la formulation « finale ». La formulation « premix » peut être stockée. Ainsi, il n'est pas nécessaire de préparer l'emulsion « premix » à chaque fois que l'on souhaite utiliser la formulation « finale », ce qui est un avantage en termes de gain de temps et de coût.

### [Procédé de préparation]

Selon un troisième aspect, l'invention concerne le procédé de préparation de la formulation définie ci-dessus.

Typiquement, on mélange d'abord les différents constituants huileux pour préparer un pré-mélange huileux pour la phase dispersée de l'émulsion, puis on le disperse dans une phase aqueuse sous l'effet d'un cisaillement.

Le procédé de préparation comprend typiquement les étapes suivantes :
(i) préparer une phase huileuse comprenant un lipide solubilisant, un lipide amphiphile, le tensioactif cationique;
(ii) préparer une phase aqueuse comprenant éventuellement le co-tensioactif;
(iii) disperser la phase huileuse dans la phase aqueuse sous l'action d'un cisaillement suffisant pour former une formulation « prémix »; puis
(iv) ajouter des séquences nucléotidiques susceptibles de moduler des mécanismes endogènes d'ARN interférence à la formulation « prémix » formée, puis
(v) récupérer la formulation ainsi formée.

### • Etape (i)

Généralement, la préparation de la phase huileuse comprend le mélange des composants huileux de la formulation (lipide solubilisant / lipide amphiphile / tensioactif cationique). Lorsque la formulation comprend un lipide susceptible de faciliter la libération cytosolique par déstabilisation de la membrane endosomale et/ou une huile, et/ou un agent d'imagerie et/ou un agent thérapeutique, ceux-ci sont généralement introduits dans la phase huileuse lors de l'étape (i).

Le mélange peut éventuellement être facilité par mise en solution d'un des constituants ou du mélange complet dans un solvant organique approprié. Le solvant organique est ensuite évaporé, pour obtenir un pré-mélange huileux homogène pour la phase dispersée.

Par ailleurs, il est préféré de réaliser le pré-mélange (étape (i)) à une température à laquelle l'ensemble des ingrédients est liquide.

### • Etape (iii)

Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une nanoémulsion, le plus souvent quelques minutes.

A la fin de l'étape (iii), on obtient généralement une nanoémulsion homogène dans laquelle :
- le diamètre moyen des gouttelettes d'huile est généralement supérieur à 10 nm et inférieur à 200 nm, de préférence entre 30 et 190 nm, et
- le potentiel zêta est supérieur à 20 mV, généralement compris entre 25 mV et 60 mV, de préférence entre 40 et 55 mV, de préférence lorsque la phase aqueuse de la formulation est une solution aqueuse de NaCl 0,15 mM.

La nanoémulsion obtenue à la fin de l'étape (iii) correspond à la formulation « prémix » définie ci-dessus.

### • Etape (iv)

L'étape (iv) permet alors de préparer la formulation utile pour la délivrance desdites séquences nucléotidiques par complexation desdites séquences nucléotidiques sur la formulation « premix » obtenue à la fin de l'étape (iii).

Généralement, les séquences nucléotidiques susceptibles de moduler des mécanismes endogènes d'ARN interférence sont ajoutées à la nanoémulsion formée et le mélange obtenu est mélangé à température ambiante, par exemple 30 minutes.

Lors de l'étape (iv), les séquences nucléotidiques susceptibles de moduler des mécanismes endogènes d'ARN interférence, porteuses de groupes phosphates chargés négativement, se lient par liaisons électrostatiques aux gouttelettes dont la surface est chargée positivement grâce aux groupes cationiques des tensioactifs cationiques. Des complexes sont ainsi formés entre les séquences nucléotidiques susceptibles de moduler des mécanismes endogènes d'ARN interférence et les gouttelettes de la nanoémulsion. Les séquences nucléotidiques susceptibles de moduler des mécanismes endogènes d'interférence par ARN sont généralement ajoutées en solution aqueuse, par exemple de l'eau exempte de nucléases, des milieux de culture cellulaire, des milieux de culture optimisés pour la transfection, notamment du milieu Opti-MEM ou des solutions tampons, notamment de l'acide 4-(2-hydroxyéthyl)-1-pipérazine éthane sulfonique (HEPES). L'étape (iv) est typiquement réalisée à température ambiante (25°C), après simple homogénéisation ou sous agitation (par exemple entre 100 et 1000 tours par minute), pendant une durée comprise entre 5 minutes et 2 heures, par exemple de l'ordre de 30 minutes.

De préférence, lors de l'étape (iv), la quantité de séquences nucléotidiques introduite est telle que le rapport entre la quantité de charges positives dues au tensioactif cationique dans la formulation « prémix » sur la quantité de charges négatives apportées par les séquences nucléotidiques introduites dans le milieu est supérieur à 8/1. L'homme du métier est à même de calculer la quantité de charges négatives apportées par les séquences nucléotidiques introduites dans le milieu, et la quantité de charges positives dues au tensioactif cationique dans la formulation « prémix ». Un tel rapport permet d'obtenir une complexation quantitative, c'est-à-dire qu'il ne reste plus de séquences nucléotidiques susceptibles de moduler des mécanismes endogènes d'ARN interférents libres dans le milieu.

L'étape (iv) peut être suivie par diverses méthodes, par exemple par :
- électrophorèse sur gel d'agarose, qui permet d'observer la migration des séquences nucléotidiques susceptibles de moduler des mécanismes endogènes d'ARN interférence. S'il y a une bonne complexation, alors les séquences nucléotidiques susceptibles de moduler des mécanismes endogènes d'ARN interférence complexées aux gouttelettes sont plus lourdes et sont visualisées dans les puits. Si la complexation est moins importante, des séquences nucléotidiques susceptibles de moduler des mécanismes endogènes d'ARN interférence libres migreront à une autre position.
- diffusion dynamique de la lumière (DLS), en observant l'impact de la complexation sur le diamètre hydrodynamique. Plus la complexation est efficace, plus le profil s'oriente vers une distribution monomodale.

A la fin de l'étape (iv), on obtient généralement une nanoémulsion homogène dans laquelle le diamètre moyen des gouttelettes d'huile est généralement supérieur à 10 nm et inférieur à 200 nm, de préférence entre 60 et 200 nm.

Avantageusement, le procédé de préparation de la formulation ne nécessite pas de modifier chimiquement les séquences nucléotidiques, et ne nécessite en particulier pas de les greffer de façon covalente à un autre composant de la formulation. Il est donc très aisé de préparer en parallèle de nombreuses formulations selon l'invention comprenant des séquences nucléotidiques.

### • Eventuelles étapes ultérieures

La formulation peut être purifiée, par exemple par purification sur colonne ou par dialyse.

Avant conditionnement, l'émulsion peut être diluée et/ou stérilisée, par exemple par filtration ou dialyse. Cette étape permet d'éliminer les éventuels agrégats qui pourraient s'être formés au cours de la préparation de l'émulsion.

L'émulsion ainsi obtenue est prête à l'emploi, le cas échéant après dilution.

### • Préparation d'une formulation comprenant un tensioactif de formule (I)

Dans le mode de réalisation dans lequel la formulation comprend un tensioactif de formule (I), la nanoémulsion utilisée pour la complexation avec les séquences nucléotidiques susceptibles de moduler des mécanismes endogènes d'ARN interférence peut être préparée par un procédé comprenant la mise en contact :
- d'une émulsion 1 comprenant une phase aqueuse continue et une phase dispersée sous forme de gouttelettes comprenant un lipide amphiphile et un tensioactif de formule (LI) suivante :

   L₁-X₁-H₁-Y₁-G₁ (LI),
- avec une émulsion 2 comprenant une phase aqueuse continue et une phase dispersée sous forme de gouttelettes comprenant un lipide amphiphile et un tensioactif de formule (LII) suivante :

   G₂-Y₂-H₂₋X₂-L₂ (LII)

   où L₁, X₁, H₁, Y₁, L₂, X₂, H₂ et Y₂ sont tels que définis ci-dessus, et G₁ et G₂ sont des groupes susceptibles de réagir pour former le groupe G tel que défini ci-dessus,
   dans des conditions permettant la réaction des tensioactifs de formules (LI) et (LII) pour former le tensioactif de formule (I) tel que défini ci-dessus, ce par quoi des liaisons covalentes entre les gouttelettes de la phase dispersée se forment.

Les phases aqueuses continues des émulsions 1 et 2 comprennent un co-tensioactif tel que défini ci-dessus. Les phases dispersées des émulsions 1 et 2 comprennent un lipide solubilisant tel que défini ci-dessus. La phase dispersée de l'émulsion 1 et/ou la phase dispersée de l'émulsion 2 comprend un tensioactif cationique tel que défini ci-dessus.

Lorsque le groupe G comprend un seul groupe G', les groupes G₁ et G₂ sont typiquement des groupes susceptibles de réagir l'un avec l'autre pour former le groupe G.

Lorsque le groupe G comprend plusieurs groupes G', on met généralement les émulsions 1 et 2 en contact avec un composé susceptible de réagir avec les tensioactifs de formules (LI) et (LII) pour former le groupe G. Ce composé comprend typiquement au moins v fonctions G'₁ susceptibles de réagir avec le groupe G₁ et w fonctions G'₂ susceptibles de réagir avec le groupe G₂.

Ainsi, dans le mode de réalisation dans lequel le groupe G répond à la formule -G'-Y₃-G'- définie ci-dessus, le procédé de préparation de la formulation comprend typiquement la mise en contact :
- d'une émulsion 1 telle que définie ci-dessus,
- et d'une émulsion 2 telle que définie ci-dessus,
- avec un composé de formule G'₁-Y₃-G'₂ dans laquelle Y₃ est tel que défini ci-dessus, G'₁ est un groupe susceptible de réagir avec G₁ pour former le premier groupe G' tel que défini ci-dessus et G'₂ est un groupe susceptible de réagir avec G₂ pour former le second groupe G' tel que défini ci-dessus (de nature identique ou différente du premier groupe G'),
dans des conditions permettant la réaction des tensioactifs de formules (LI) et (LII) et du composé de formule G'₁-Y₃-G'₂ pour former le tensioactif de formule (I) dans lequel le groupe G répond à la formule -G'-Y₃-G'- définie ci-dessus, ce par quoi des liaisons covalentes entre les gouttelettes de la phase dispersée se forment.

De même, dans le mode de réalisation défini ci-dessus dans lequel le groupe G est un dendrimère comprenant (v+w) groupes G', le procédé de préparation de la formulation comprend typiquement la mise en contact :
- d'une émulsion 1 telle que définie ci-dessus,
- et d'une émulsion 2 telle que définie ci-dessus,
- avec un dendrimère de formule (G'₁)ᵥ-Y₄-(G'₂)_{w} dans laquelle v et w et sont tels que définis ci-dessus, G'₁ est indépendamment un groupe susceptible de réagir avec G₁ pour former un groupe G' tel que défini ci-dessus et G'₂ est indépendamment un groupe susceptible de réagir avec G₂ pour former un groupe G' tel que défini ci-dessus (chaque G' étant de nature identique ou différente des autres groupes G') et Y₄ est le squelette d'un dendrimère,
dans des conditions permettant la réaction des tensioactifs de formules (LI) et (LII) et du dendrimère de formule (G'₁)ᵥ-Y₄-(G'₂)_{w} pour former le tensioactif de formule (I) dans lequel le groupe G est un dendrimère comprenant (v+w) groupes G', ce par quoi des liaisons covalentes entre les gouttelettes de la phase dispersée se forment.
Par exemple, pour former un groupe G ayant la formule (XXX), (XXXI), (XXXII) et (XXXIII), le composé de formule (G'₁)ᵥ-Y₄-(G'₂)_{w} peut respectivement avoir une des formules (XXX'), (XXXI'), (XXXII') et (XXXIII') suivantes : 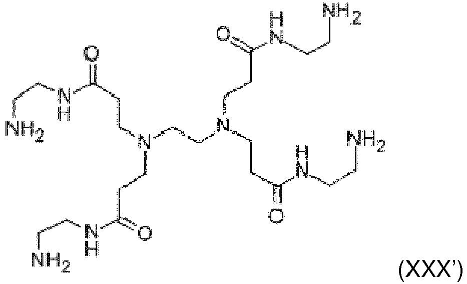 (dans laquelle G'₁ et G'₂ -représentent NH₂ et v et w représentent 2), 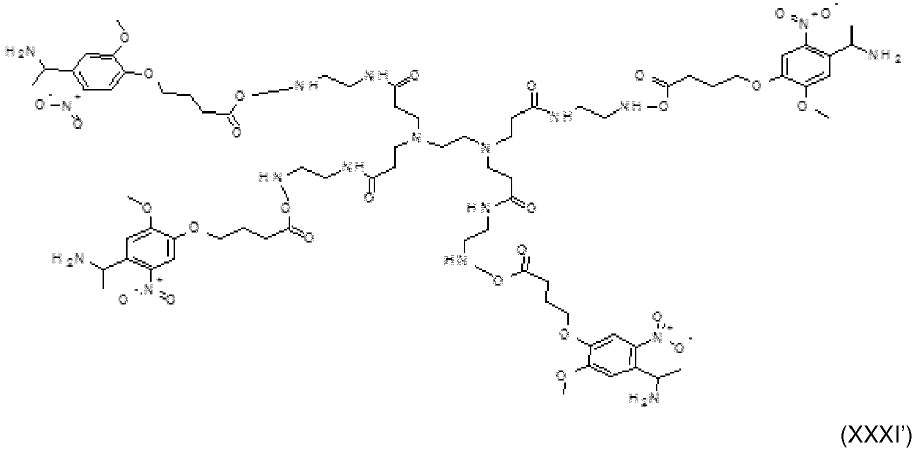 (dans laquelle G'₁ et G'₂ -représentent NH₂ et v et w représentent 2), 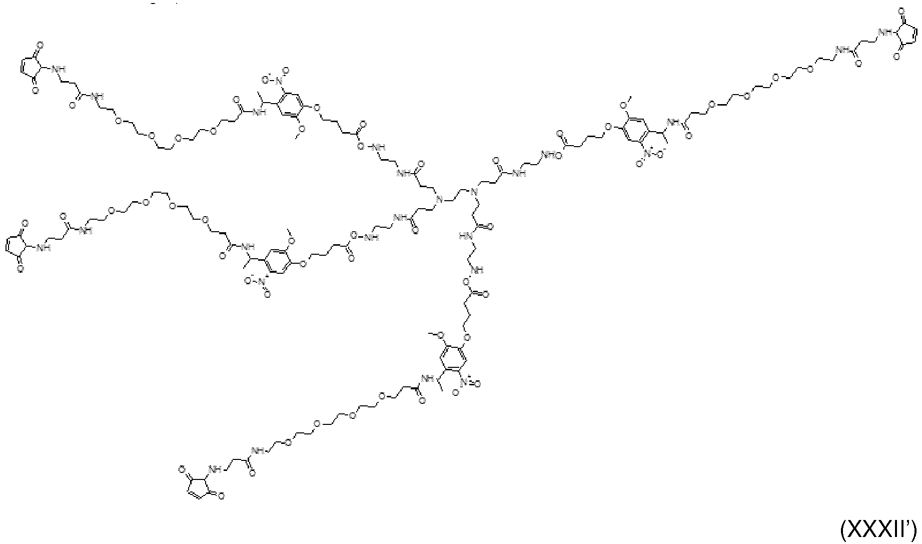 (dans laquelle G'₁ et G'₂ -représentent 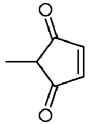 et v et w représentent 2), 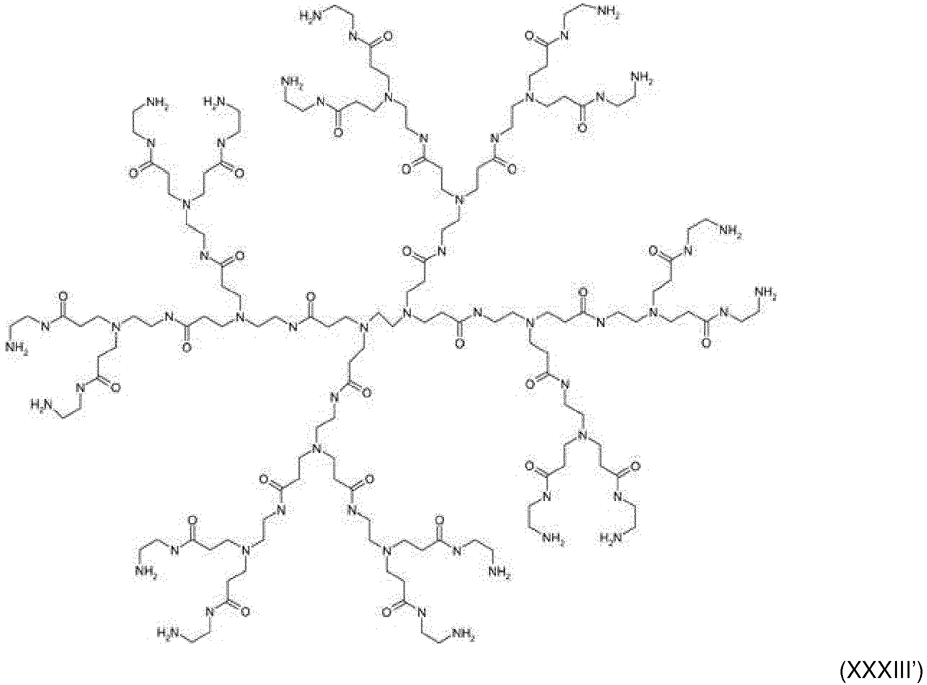 (dans laquelle G'₁ et G'₂ -représentent NH₂ et v et w représentent 8).

Au vu de ses connaissances générales en chimie, l'homme du métier est à même de choisir la nature des groupes G'₁, G'₂, Y₃, Y₄, G₁ et G₂ à utiliser pour former le groupe G et les conditions permettant la réaction. Les réactions de chimie organique usuelles peuvent être suivies, notamment celles décrites dans « Comprehensive Organic Transformations: A Guide to Functional Group Préparations » de Richard C. Larock édité par John Wiley & Sons Inc, et les références qui y sont citées. Ainsi, les exemples de groupes G₁ et G₂ ci-dessous sont cités à titre illustratif et non limitatif.

Typiquement, lorsque le groupe G est constitué d'un groupe G', les groupes G₁ et G₂ des composés de formules (LI) et (LII) peuvent par exemple être choisis comme suit :
- G₁ représente un thiol (-SH) et G₂ représente :
   - soit un maléimide, un tensioactif de formule (I) dans lequel G comprend un groupe G' représentant un groupe de formule (XIV) où A₁₀₂ représente N étant alors formé,
   - soit une vinylsulfone, un tensioactif de formule (I) dans lequel G comprend un groupe G' représentant un groupe de formule (XVI) étant alors formé,
   - soit un groupe -S-S-pyridinyle ou -SH, un tensioactif de formule (I) dans lequel G comprend un groupe G' représentant un groupe de formule (XV) étant alors formé,
- G₁ représente un hydroxyle et G₂ représente -COOH ou un ester activé, un tensioactif de formule (I) dans lequel G comprend un groupe G' représentant un groupe de formule (XXIII) étant alors formé,
- G₁ représente une amine -NH₂ et G₂ représente -COOH ou un ester activé, un tensioactif de formule (I) dans lequel G comprend un groupe G' représentant un groupe de formule (XI) étant alors formé,
- G₁ représente un hydroxyle et G₂ représente un carbonate activé, un tensioactif de formule (I) dans lequel G comprend un groupe G' représentant un groupe de formule (XIX) étant alors formé,
- G₁ représente une amine -NH₂ et G₂ représente un carbonate activé, un tensioactif de formule (I) dans lequel G comprend un groupe G' représentant un groupe de formule (XII) étant alors formé,
- G₁ représente une amine -NH₂ et G₂ représente un aldéhyde -CHO, un tensioactif de formule (I) dans lequel G comprend un groupe G' représentant un groupe de formule (XXI) étant alors formé,
- G₁ représente un hydrazide de formule -(C=O)-NH-NH₂ et G₂ représente un groupe - (C=O)-R10₂, un tensioactif de formule (I) dans lequel G comprend un groupe G' représentant un groupe de formule (XIII) étant alors formé,
- G₁ représente un alcyne et G₂ représente un azoture, un tensioactif de formule (I) dans lequel G comprend un groupe G' représentant un groupe de formule (XVIII) étant alors formé,
- G₁ représente un cyclooctyne et G₂ représente un azoture, un tensioactif de formule (I) dans lequel G comprend un groupe G' représentant un groupe de formule (XVIII') étant alors formé,
- G₁ représente un furane et G₂ représente un maléimide, un tensioactif de formule (I) dans lequel G comprend un groupe G' représentant un groupe de formule (XVII) étant alors formé,
- G₁ représente un aldéhyde et G₂ représente une amine, un tensioactif de formule (I) dans lequel G comprend un groupe G' représentant un groupe de formule (XXI) étant alors formé,
- G₁ représente un phosphate de formule -O-P(=O)(OH)₂ et G₂ représente un hydroxyle, un tensioactif de formule (I) dans lequel G comprend un groupe G' représentant un groupe de formule (XXII) étant alors formé,
- G₁ représente un bon groupe partant LG et G₂ représente un groupe de formule suivante 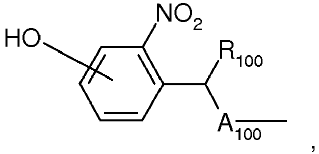 un tensioactif de formule (I) dans lequel G comprend un groupe G' représentant un groupe de formule (XXIV) dans laquelle A₁₀₁ représente O étant alors formé,
- G₁ représente un hydroxyle ou une amine -NH₂ et G₂ représente un groupe de formule suivante 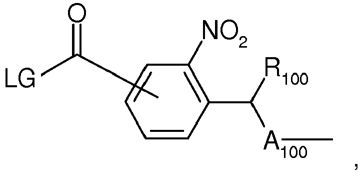 un tensioactif de formule (I) dans lequel G comprend un groupe G' représentant un groupe de formule (XXIV) dans laquelle A₁₀₁ représente respectivement -O-(CO)- ou -NH-(CO) étant alors formé,
- G₁ représente un bon groupe partant LG et G₂ représente un hydroxyle, un tensioactif de formule (I) dans lequel G comprend un groupe G' représentant un groupe de formule (XXV) étant alors formé,
- G₁ représente un bon groupe partant LG et G₂ représente une amine -NH₂, un tensioactif de formule (I) dans lequel G comprend un groupe G' représentant un groupe de formule (XXVI) étant alors formé,
- G₁ représente une oxyamine -O-NH₂ et G₂ représente un aldéhyde, un tensioactif de formule (I) dans lequel G comprend un groupe G' représentant un groupe de formule (XXVII) étant alors formé.

Lorsque le groupe G comprend plusieurs groupes G', le choix des groupes réagissant ensemble : G'₁ et G₁ d'une part et G'₂ et G₂ d'autre part, peut être effectué de la même façon, en remplaçant les groupes G₁ ou G₂ dans les exemples mentionnés ci-dessus par G'₁ ou G'₂.

Les émulsions 1 et 2 mises en oeuvre dans le procédé peuvent être préparées par le premier procédé susmentionné comprenant les étapes (i), (ii), (iii) et (v) (en n'effectuant pas l'étape (iv) d'ajout des séquences nucléotidiques susceptibles de moduler des mécanismes endogènes d'ARN interférence).

### [Utilisation]

La formulation est avantageusement stable et peu toxique. Elle peut être utilisée comme agent de transfection et/ou comme système de délivrance de séquences nucléotidiques susceptibles de moduler des mécanismes endogènes d'ARN interférence.

Lorsque la formulation comprend un agent d'imagerie, il est avantageusement possible de suivre la délivrance des séquences nucléotidiques susceptibles de moduler des mécanismes endogènes d'ARN interférence et/ou la transfection des cellules, par exemple par suivi de la fluorescence lorsque l'agent d'imagerie est un fluorophore lipophile.

### • Comme agent de transfection

Selon un quatrième aspect, l'invention concerne une méthode d'introduction de séquences nucléotidiques susceptibles de moduler des mécanismes endogènes d'ARN interférence dans une cellule eucaryote, comprenant la mise en contact de la cellule eucaryote avec la formulation selon l'invention. Cette méthode est une méthode de transfection.

La méthode d'introduction peut être réalisée in vitro. Dans ce cas, généralement, la mise en contact de la cellule eucaryote avec la formulation selon l'invention a lieu dans une solution tampon, par exemple un milieu OptiMEM®. La mise en contact dure généralement entre 8 et 96 heures, typiquement de l'ordre de 72 heures, à une température de l'ordre de 37°C.

Généralement, après l'étape de mise en contact, les cellules sont récupérées.

Non seulement les gouttelettes de la formulation selon l'invention permettent de transfecter efficacement les cellules, mais en plus elles sont fonctionnellement actives et permettent d'éteindre l'expression du gène d'intérêt.

Sans vouloir être liés à une théorie particulière, une fois que les gouttelettes de la nanoémulsion selon l'invention ont transfecté la cellule, l'échappement endosomal libérerait les gouttelettes et les séquences nucléotidiques susceptibles de moduler des mécanismes endogènes d'ARN interférence dans le cytoplasme, donc dans le site d'action des séquences nucléotidiques susceptibles de moduler des mécanismes endogènes d'interférence par ARN, ce qui éviterait la dégradation de ces séquences nucléotidiques dans le lysosome, leur permettant d'interférer avec l'ARNm cible et donc d'éteindre ainsi l'expression du gène d'intérêt.

Dans un mode de réalisation, la formulation comprend un co-tensioactif greffé par un ligand biologique de ciblage. Ce mode de réalisation permet avantageusement l'introduction de séquences nucléotidiques susceptibles de moduler des mécanismes endogènes d'ARN interférence dans des cellules eucaryotes usuellement résistantes à la transfection, c'est-à-dire connues comme étant difficiles à transfecter, notamment des cellules souches, des cellules primaires (par exemple des fibroblastes humains ou des cellules endothéliales humaines) ou des lignées cellulaires lymphocytaires (par exemple de type Jurkat) ou de neuroblastomes (par exemple de type SK-N-SH). Le ligand biologique de ciblage permet en effet de faciliter la transfection de cellules usuellement résistantes à la transfection par la formulation.

### • pour la délivrance de séquences nucléotidiques susceptibles de moduler des mécanismes endogènes d'ARN interférence

La formulation selon l'invention est un système de délivrance in vitro ou in vivo de séquences nucléotidiques susceptibles de moduler des mécanismes endogènes d'ARN interférence.

Ainsi, selon un cinquième aspect, l'invention concerne la formulation définie ci-dessus pour son utilisation pour la prévention et/ou le traitement d'une maladie.

La maladie est typiquement une maladie pour laquelle une extinction d'un ou plusieurs gènes est recherchée, telle que :
- les maladies oculaires, notamment la dégénérescence maculaire liée à l'âge (DMLA), le glaucome ou la Pachyonychia congenitale.
- le cancer, les tumeurs solides, les métastases mais également les leucémies myéloïdes chroniques.
- les troubles rénaux, notamment après transplantation ou insuffisance rénale aigue.
- l'hypercholestérolémie.
- les maladies virales liées notamment à une infection, par le virus de l'hépatite C (VHC), par le virus de l'immunodéficience humaine (VIH) et par le virus respiratoire syncytial (VRS).

Avantageusement, comme explicité ci-dessus, la formulation selon l'invention protège les séquences nucléotidiques susceptibles de moduler des mécanismes endogènes d'ARN interférence du corps du patient (et de ces protéines) auquel la formulation est administrée (notamment en évitant son métabolisme prématuré).

La formulation peut comprendre un agent thérapeutique permettant de traiter la maladie, ce qui permet de bénéficier d'un double effet thérapeutique : celui induit par les séquences nucléotidiques susceptibles de moduler des mécanismes endogènes d'ARN interférence et celui induit par l'agent thérapeutique.

Du fait qu'elle peut être préparée exclusivement à partir de constituants approuvés pour l'homme, elle est intéressante pour une administration par voie parentérale. Cependant, il est également possible d'envisager une administration par d'autres voies, notamment par voie orale, par voie topique, ou par voie ophtalmologique.

Une méthode de prévention et/ou de traitement d'une maladie comprenant l'administration chez un mammifère, de préférence un humain, qui en a besoin d'une quantité efficace de la formulation telle que définie ci-dessus est également un des objets de la présente invention.

### [Définitions]

Dans le cadre de la présente demande, on entend par le terme « nanoémulsion » une composition présentant au moins deux phases, généralement une phase huileuse et une phase aqueuse, dans laquelle la taille moyenne de la phase dispersée est inférieure à 1 micron, de préférence de 10 à 500 nm et en particulier de 20 à 200 nm, et tout préférentiellement de 50 à 200 nm (voir article C. Solans, P. Izquierdo, J. Nolla, N. Azemar and M. J. Garcia-Celma, Curr Opin Colloid In, 2005, 10, 102-110).

Au sens de la présente demande, l'expression « phase dispersée » signifie les gouttelettes comprenant l'éventuelle huile / le(s) tensioactif(s) cationique(s) / le lipide solubilisant/ le lipide amphiphile/ le co-tensioactif/ l'éventuel tensiactif de formule (I)/ l'éventuel lipide fusogène / l'éventuel agent d'imagerie / l'éventuel agent thérapeutique / les éventuelles séquences nucléotidiques susceptibles de moduler des mécanismes endogènes d'ARN interférents. La phase dispersée est généralement exempte de phase aqueuse.

Le terme « gouttelette » englobe à la fois les gouttelettes d'huile liquide proprement dites ainsi que les particules solides issues d'émulsions de type huile-dans-eau dans lesquelles la phase dispersée est solide. Dans ce dernier cas, on parle souvent aussi d'émulsion solide.

Le terme « lipide » désigne dans le cadre de cet exposé l'ensemble des corps gras ou des substances contenant des acides gras présents dans les graisses d'origine animales et dans les huiles végétales. Ce sont des molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on citera en particulier la phosphatidylcholine, la phosphatidyl éthanolamine, la phophatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un tensioactif pour abaisser davantage l'énergie de l'interface.

On entend par le terme « chaîne hydrocarbonée » désigner une chaîne composée d'atomes de carbone et d'hydrogène, saturée ou insaturée (double ou triple liaison). Les chaînes hydrocarbonées préférées sont les alkyle ou alcényle.

On entend par le terme « alkylène » désigner un groupe divalent aliphatique hydrocarboné, saturé, linéaire ou ramifié, de préférence linéaire.

On entend par « radical cyclique » un radical issu d'un cycle. Par exemple, un radical phénylène est un radical divalent issu d'un groupe benzène. Par « cycle », on entend un carbocycle ou un hétérocycle, saturé, insaturé ou aromatique (aryle ou hétéroaryle),
- un carbocycle : un cycle composé d'atomes de carbone, saturé (les carbocycles saturés préférés étant notamment un cycloalkyle, tel que un cyclopentyle ou un cyclohexyle), insaturé (par exemple un cyclohexène) ou aromatique (c'est-à-dire un phényle),
- un hétérocycle : un groupe cyclique comprenant, sauf mention contraire, de 5 à 6 atomes, et comprenant un ou plusieurs hétéroatomes choisis parmi O, N et/ou S. Ledit hétérocycle peut être saturé ou partiellement insaturé et comprendre une ou plusieurs doubles liaisons. On parle alors de groupe hétérocycloalkyle. Il peut également être aromatique, comprenant, sauf mention contraire, de 5 à 6 atomes et représenter alors un groupe hétéroaryle.
   - à titre d'hétérocycle non aromatique ou hétérocycloalkyle, on peut citer, le pyrazolidinyle, l'imidazolidinyle, le tétrahydrothiophényle, le dithiolanyle, le thiazolidinyle, le tétrahydropyranyle, le dioxanyle, le morpholinyle, le pipéridyle, le pipérazinyle, le tétrahydrothiopyranyle, le thiomorpholinyle, le dihydrofuranyle, le 2-imidazolinyle, le 2,-3-pyrrolinyle, le pyrazolinyle, le dihydrothiophényle, le dihydropyranyle, le pyranyle, le tétrahydropyridyle, le dihydropyridyle, le tétrahydropyrinidinyle, le dihydrothiopyranyle, l'isoxazolidinyle,
   - à titre d'hétéroaryle, on peut notamment citer les groupes représentatifs suivants : furyle, imidazolyle, isoxazolyle, isothiazolyle, oxadiazolyle, 1,2,4-oxadiazolyle, 1,3,4-oxadiazolyle, pyrazinyle, pyridazinyle, pyrazolyle, pyridyle, pyrimidinyle, pyrrolyle, 1,3,4-thiadiazolyle, 1,2,3-thiadiazolyle, thiazolyle, thiényle, triazolyle, 1,2,3-triazolyle et 1,2,4-triazolyle.

Par « ester activé », on entend un groupe de formule -CO-LG, et par « carbonate activé », on entend un groupe de formule -O-CO-LG où LG est un bon groupe partant notamment choisi parmi un chlore, un imidazolyle, un pentafluorophénolate, un pentachlorophénolate, un 2,4,5-trichlorophénolate, 2,4,6-trichlorophénolate, un groupe -O-succinimidyle, -O-benzotriazolyle, -O-(7-aza-benzotriazolyle) et -O-(4-nitrophényle).

L'invention sera décrite plus en détail au moyen de la figure en annexe et des exemples qui suivent.

### FIGURES

La figure 1 représente un schéma illustrant la structure coeur/couronne d'une gouttelette d'une formulation selon l'invention (formulation « finale » avec une séquence nucléotidique susceptible de moduler des mécanismes endogènes d'ARN interférence). 1 représente la phase aqueuse continue, 2 la couronne (en partie) de la gouttelette et 3 le coeur (en partie) de la gouttelette. Dans la couronne 2, sont illustrés : le lipide amphiphile (par exemple phospholipide neutre tel que la lécithine), le tensioactif cationique (phospholipide cationique DOTAP par exemple), le co-tensioactif dont la chaîne poly(oxyde d'éthylène) se repliant est représentée dans la phase aqueuse et la séquence nucléotidique double brin, et susceptible de moduler des mécanismes endogènes d'ARN interférence (siARN par exemple).
La figure 2 représente un gel d'électrophorèse révélé à l'UV réalisé après complexation de siARN avec la formulation B10 avec des concentrations précisées au tableau 8. L'échelle et les siARN (témoin) sont à gauche.
La figure 3 donne les résultats de quantité de SiARN (ng) obtenus par traitement des données de l'électrophorèse sur le logiciel ImageJ du gel d'électrophorèse révélé à l'UV de la figure 2.
La figure 4 représente l'intensité obtenue sur un appareil ZetaSizer Malvern en fonction du diamètre hydrodynamique en nm pour le siARN libre (comparaison) et pour trois formulations selon l'invention obtenues par complexation avec un rapport N/P : quantité de charges positives dues au tensioactif cationique dans la formulation « prémix » (dues à l'azote chargé, d'où le N de N/P), sur la quantité de charges négatives apportée par les siARN (dues au phosphore du SiARN, d'où le P de N/P) de 4/1, de 8/1 ou de 16/1.
La figure 5 représente un gel d'électrophorèse révélé à l'UV avec, de gauche à droite :
   - l'échelle,
   - le siARN libre (témoin)
   puis des migrations obtenues par complexation de siARN :
   - avec la formulation B1 (exemple comparatif) (pas de complexation, les siARN sont libres),
   - avec la formulation B6 avec un rapport quantité de charges positives dues au tensioactif cationique dans la formulation « prémix » sur la quantité de charge négative apportée par les siARN de 8/1 (complexation quantitative),
   - avec la formulation B6 avec un rapport quantité de charges positives dues au tensioactif cationique dans la formulation « prémix » sur la quantité de charge négative apportée par les siARN de 8/1 (complexation quantitative),
   - avec la formulation B10 avec un rapport quantité de charges positives dues au tensioactif cationique dans la formulation « prémix » sur la quantité de charge négative apportée par les siARN de 8/1 (complexation quantitative),
   - avec de la lipofectamine (exemple comparatif) (complexation incomplète).

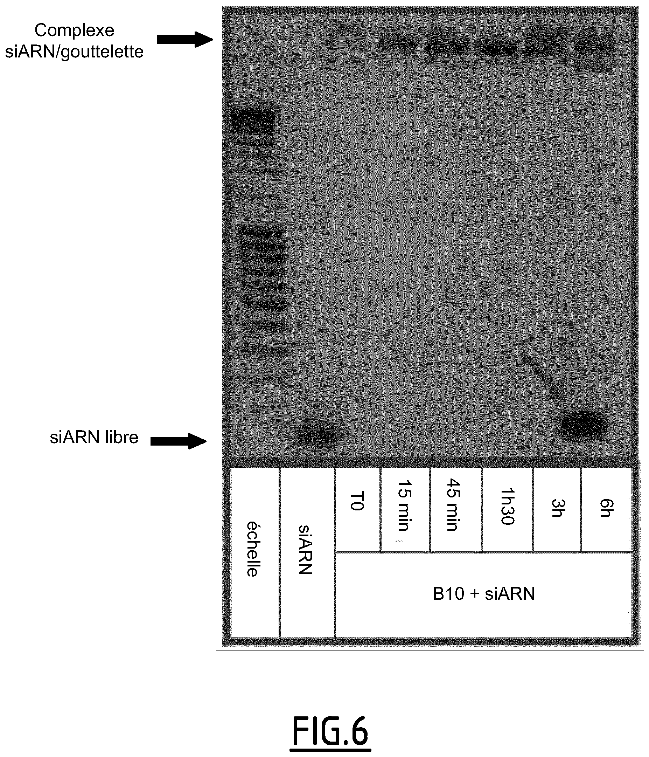

La figure 6 représente un gel d'électrophorèse révélé à l'UV d'un complexe siARN/formulation B10 juste après complexation (T0) puis 15 min, 45 min, 1h30, 3h et 6h après complexation. L'échelle et les siARN (témoin) sont à gauche.

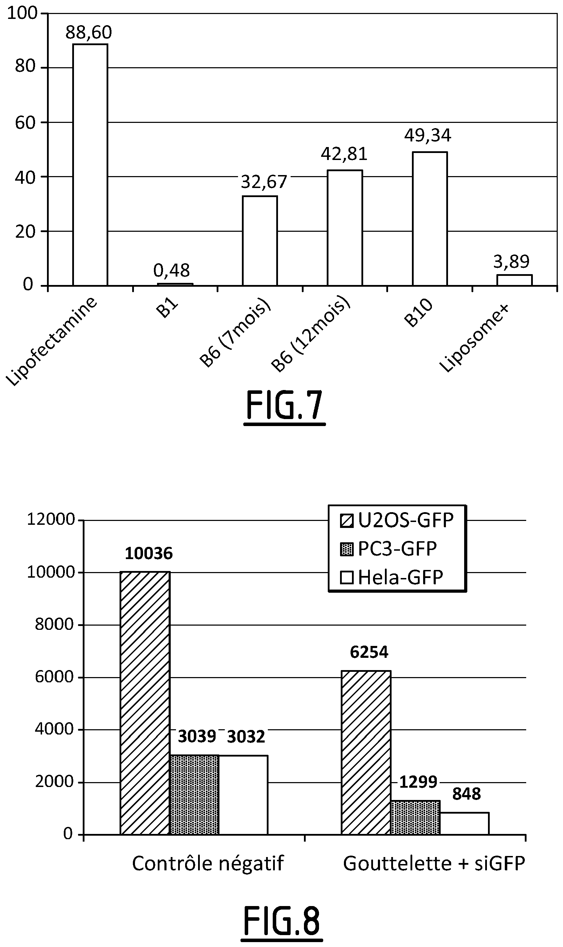

La figure 7 représente la diminution de l'intensité de fluorescence FITC en % en transfectant les lignées cellulaires avec :
   - de la Lipofectamine RNAimax (agent commercial),
   - le complexe siARN/formulation B1,
   - avec le complexe siARN/formulation B6, la formulation ayant été conservée 7 mois à température ambiante préalablement à la complexation,
   - avec le complexe siARN/formulation B6, la formulation ayant été conservée 12 mois à température ambiante préalablement à la complexation,
   - avec le complexe siARN/formulation B10, la formulation ayant été conservée 12 mois à température ambiante préalablement à la complexation,
   - avec un complexe siARN / formulation de liposomes cationiques comprenant du DOTAP (58% wt), du DOPE (18% wt), du cholestérol (2% wt) et du DSPE-PEG3000 (22% wt)) (comparatif).
La figure 8 représente l'intensité de fluorescence (FITC) pour 3 lignées cellulaires surexprimant expérimentalement la protéine fluorescente GFP (pour green fluorescent protein) : U2OS, PC3 et Hela pour le complexe Si/ARN formulation B10, le siARN appelé siGFP étant ciblé contre l'ARNm codant pour la protéine GFP, et pour le contrôle négatif (cellules incubées avec une formulation B10 complexée avec un siRNA contrôle négatif, c'est-à-dire « inerte » sans effet sur le transcriptome de la cellule appelé siAllStar).

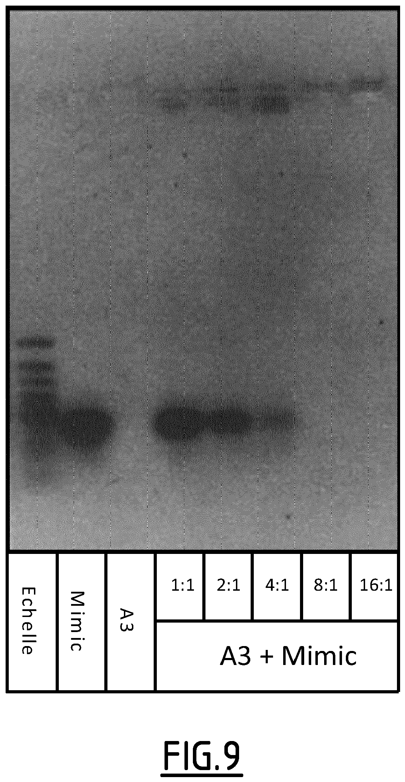

La figure 9 représente un gel d'électrophorèse révélé à l'UV réalisé après complexation de microARN synthétique de type mimic avec la formulation A3 avec des concentrations précisées au tableau 9. L'échelle et les microARN Mimic (témoin) et la formulation prémix A3 non complexée sont à gauche. Les formulations finales issues de la complexation de la formulation prémix A3 et des Mimic à cinq rapports N/P différents sont à droite.

### EXEMPLES

### • Préparation et composition de formulations « premix » avant complexation aux siARN

La phase aqueuse utilisée est une solution tampon PBS 1X.

Les fournisseurs des composés sont les suivants :
Lipoid S75-3 : Lipoid
Lipoid S75 : Lipoid
Lipoid S100-3 : Lipoid
DOTAP : Avanti Polar
DOPE : Avanti Polar
MyrjS40 : Croda
Suppocire NB : Gattefossé
Huile de soja : Croda

Le diamètre hydrodynamique des gouttelettes des formulations ainsi que leur potentiel zeta ont été mesurés par diffusion quasi-élastique de la lumière par un appareil de type ZetaSizer, Malvern. Le diamètre hydrodynamique des gouttelettes a été mesuré dans une solution de PBS 0,1X, le potentiel zeta dans une solution aqueuse de NaCl 0,15 mM.

Seize formulations différentes ont été réalisées, dont les compositions sont indiquées dans les tableaux 1 à 5.

**Tableau 1**

| | | **A1 (comp.)** | **A2** | **A3** |
|---|---|---|---|---|
| **COURONNE** | **Lipide amphiphile : Lipoid** | S75-3 | S75-3 | S75-3 |
| | ***% wt Lipoid* / *gouttelette*** | 35,29 | 8,83 | 3,53 |
| | *% wt Lipoid* /*couronne sans PEG* | 100 | 25 | 10 |
| | *% wt Lipoid l couronne* | 46,15 | 11,54 | 4,62 |
| | *% mol. Lipoid l couronne* | 70,02 | 16,86 | 6,74 |
| | **Tensioactif cationique DOTAP** | X | DOTAP | DOTAP |
| | ***% wt DO TAP* / *gouttelette*** | 0 | 26,47 | 26,47 |
| | *% wt DOTAP* /*couronne sans PEG* | 0 | 75 | 75 |
| | *% wt DOTAP* / *couronne* | 0 | 34,61 | 34,61 |
| | ***% mol. DOTAP* / *couronne*** | **0** | **54,28** | **54,24** |
| | **Co-tensioactif** | Myrj S40 | Myrj S40 | Myrj S40 |
| | ***% wt co-tensioactif*/ *gouttelette*** | 41,17 | 41,17 | 41,17 |
| | *% wt (co-tensioactiof)* / *couronne* | 53,85 | 53,85 | 53,85 |
| | ***% mol. (co-tensioactif)* / *couronne*** | **29,98** | **28,86** | **28,84** |
| | **Lipide fusogène DOPE** | X | X | DOPE |
| | ***% wt DOPE* / *gouttelette*** | 0 | 0 | 5,29 |
| | *% wt DOPE* /*couronne sans PEG* | 0 | 0 | 15 |
| | *% wt DOPE couronne* | 0 | 0 | 6,92 |
| | *% mol. DOPE l couronne* | 0 | 0 | 10,18 |
| **COEUR** | **Lipide solubilisant** | Suppocire NB | Suppocire NB | Suppocire NB |
| | *% wf lipide solubilisant*/*coeur* | 75 | 75 | 75 |
| | ***% wt lipide solubilisant* / *gouttelette*** | 17,65 | 17,65 | 17,65 |
| | **Huile** | Huile de soja | Huile de soja | Huile de soja |
| | *% wt huile* / *coeur* | 25 | 25 | 25 |
| | ***% wt huile* / *gouttelette*** | 5,88 | 5,88 | 5,88 |
| | ***% wt coeur* / *gouttelette*** | | 23,53 | 23,53 |
| | ***% mol. coeur* / *gouttelette*** | | 33,35 | 33,35 |
| | **Formulation (nombre de répétition)** | 3 | 4 | 3 |
| **RESULTAT** | **Diamètre hydrodynamique (nm) - average** | 124,9 | 49,1 | 44,48 |
| | **ZP (mV)_dans NaCl 0,15 mM** | -26 | 25,38 | 30,87 |
| | **Stabilité** | ok | ok | ok |
| | **Complexation (%)** | 0 | 100 | 100 |
| | **Efficacité d'extinction (%)** | 0 | 72,88 | 75,06 |

**Tableau 2**

| | | **B1 (comp.)** | **B2** | **B3** |
|---|---|---|---|---|
| **COURONNE** | **Lipide amphiphile : Lipoid** | S75-3 | S75 | S100-3 |
| | ***% wt Lipoid* / *gouttelette*** | 8,75 | 4,25 | 4,25 |
| | *% wt Lipoid* /*couronne sans PEG* | 100 | 50 | 50 |
| | *% wt Lipoid l couronne* | 15,98 | 7,99 | 7,99 |
| | *% mol. Lipoid* / *couronne* | 34,14 | 17,89 | 17,89 |
| | **Tensioactif cationique DOTAP** | X | DOTAP | DOTAP |
| | ***% wt DO TAP* / *gouttelette*** | 0 | 4,25 | 4,25 |
| | *% wt DOTAP* / *couronne sans PEG* | 0 | 50 | 50 |
| | *% wt DOTAP* / *couronne* | 0 | 7,99 | 7,99 |
| | ***% mol. DOTAP* / *couronne*** | **0** | **17,85** | **17,85** |
| | **Co-tensioactif** | Myrj S40 | Myrj S40 | Myrj S40 |
| | ***% wt_co-tensioactif* / *gouttelette*** | 46 | 46 | 46 |
| | *% wt (co-tensioactiof)* / *couronne* | 84,02 | 84,02 | 84,02 |
| | ***% mol. (co-tensioactif)* / *couronne*** | **65,86** | **64,27** | **64,27** |
| | **Lipide fusogène DOPE** | X | X | X |
| | ***% wt DOPE* / *gouttelette*** | 0 | 0 | 0 |
| | *% wt DOPE* /*couronne sans PEG* | 0 | 0 | 0 |
| | *% wt DOPE* /*couronne* | 0 | 0 | 0 |
| | *% mol. DOPE l couronne* | 0 | 0 | 0 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 33,94 | 33,94 | 33,94 |
| | **Huile** | Huile de soja | Huile de soja | Huile de soja |
| | *% wt huile* / *coeur* | 25 | 25 | 25 |
| | ***% wt huile* / *gouttelette*** | 11,31 | 11,31 | 11,31 |
| | ***% wt coeur* / *gouttelette*** | | 45,25 | 45,25 |
| | ***% mol. coeur* / *gouttelette*** | | | |
| | **Formulation (nombre de répétition)** | 6 | 2 | 2 |
| **RESULTAT** | **Diamètre hydrodynamique (nm) - average** | 59,51 | 42,11 | 61,43 |
| | **ZP (mV)_dans NaCl 0,15 mM** | -21,8 | 21,4 | 6,72 |
| | **Stabilité** | ok | ok | ok |
| | **Complexation (%)** | 0 | ND | ND |
| | **Efficacité d'extinction (%)** | 0 | 0 | 0 |

**Tableau 3**

| | | **B4 (comp.)** | **B5** | **B6** |
|---|---|---|---|---|
| **COURONNE** | **Lipide amphiphile : Lipoid** | S75-3 | S75-3 | S75-3 |
| | ***% wt Lipoid* / *gouttelette*** | 6,58 | 2,19 | 2,84 |
| | *% wt Lipoid* / *couronne sans PEG* | 100 | 25 | 25 |
| | *% wt Lipoid l couronne* | 14,29 | 4 | 6,9 |
| | *% mol. Lipoid l couronne* | 31,24 | 8,39 | 12,39 |
| | **Tensioactif cationique DOTAP** | X | DOTAP | DOTAP |
| | ***% wt DO TAP* / *gouttelette*** | 0 | 6,56 | 8,52 |
| | *% wt DOTAP* / *couronne sans PEG* | 0 | 75 | 75 |
| | *% wt DOTAP* / *couronne* | 0 | 11,99 | 20,67 |
| | ***% mol. DOTAP* / *couronne*** | **0** | **26,99** | **39,87** |
| | **Co-tensioactif** | Myrj S40 | Myrj S40 | Myrj S40 |
| | ***% wt_co-tensioactif* / *gouttelette*** | 39,48 | 46 | 29,87 |
| | *% wt (co-tensioactiof)* / *couronne* | 85,71 | 84,02 | 72,43 |
| | ***% mol. (co-tensioactif)* / *couronne*** | **68,76** | **64,63** | **47,74** |
| | **Lipide fusogène DOPE** | X | X | X |
| | *%* ***wt DOPE* / *gouttelette*** | 0 | 0 | 0 |
| | *% wt DOPE l couronne sans PEG* | 0 | 0 | 0 |
| | *% wt DOPE l couronne* | 0 | 0 | 0 |
| | *% mol. DOPE l couronne* | 0 | 0 | 0 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 40,46 | 33,94 | 44,07 |
| | **Huile** | Huile de soja | Huile de soja | Huile de soja |
| | *% wt huile* / *coeur* | 25 | 25 | 25 |
| | ***% wt huile* / *gouttelettes*** | 13,49 | 11,31 | 14,69 |
| | ***% wt coeur* / *gouttelette*** | | 45,25 | 58,76 |
| | ***% mol. caeur l gouttelette*** | | | 73,99 |
| | **Formulation (nombre de répétition)** | 3 | 4 | 6 |
| **RESULTAT** | **Diamètre hydrodynamique (nm) - average** | 84,88 | 56,68 | 86,77 |
| | **ZP (mV)_dans NaCl 0,15 mM** | -18,89 | 26,51 | 36,38 |
| | **Stabilité** | ok | ok | ok |
| | **Complexation (%)** | 0 | 100 | 100 |
| | **Efficacité d'extinction (%)** | 0 | 0 | 42,81 |

**Tableau 4**

| | | **B9 (Comp.)** | **B10** |
|---|---|---|---|
| **COURONNE** | **Lipide amphiphile : Lipoid** | S75-3 | S75-3 |
| | ***% wt Lipoid* / *gouttelette*** | 0 | 1,71 |
| | *% wt Lipoid* /*couronne sans PEG* | 0 | 15 |
| | *% wt Lipoid l couronne* | 0 | 4,14 |
| | *% mol. Lipoid l couronne* | 0 | 7,43 |
| | **Tensioactif cationique DOTAP** | DOTAP | DOTAP |
| | ***% wt DO TAP* / *gouttelette*** | 8,25 | 8,25 |
| | *% wt DOTAP* / *couronne sans PEG* | 75 | 75 |
| | *% wt DOTAP l couronne* | 20,67 | 20,67 |
| | ***%** mol.* ***DO TAP* / *couronne*** | **39,87** | **39,87** |
| | **Co-tensioactif** | Myrj S40 | Myrj S40 |
| | *%* ***wt_co-tensioactif* / *gouttelette*** | 29,87 | 29,87 |
| | *% wt (co-tensioactiof)* / *couronne* | 72,43 | 72,43 |
| | ***% mol. (co-tensioactif)* / *couronne*** | **47,74** | **47,74** |
| | **Lipide fusogène DOPE** | DOPE | DOPE |
| | *%* ***wt DOPE* / *gouttelette*** | 2,84 | 1,71 |
| | *% wt DOPE* / *couronne sans PEG* | 25 | 10 |
| | *% wt DOPE couronne* | 6,9 | 2,76 |
| | *% mol. DOPE l couronne* | 10,18 | 4,99 |
| **COEUR** | **Lipide solubilisant** | Suppocire NB | Suppocire NB |
| | *% wt lipide solubilisant*/*coeur* | 75 | 75 |
| | ***% wt lipide solubilisant* / *gouttelette*** | 44,07 | 44,07 |
| | **Huile** | Huile de soja | Huile de soja |
| | *% wt huile* / *coeur* | 25 | 25 |
| | ***% wt huile* / *gouttelettes*** | 14,69 | 14,69 |
| | ***% wt coeur* / *gouttelette*** | | 58,76 |
| | ***% mol. caeur l gouttelette*** | | 73,74 |
| | **Formulation (nombre de répétition)** | 1 mauvaise formulation | 5 |
| **RESULTAT** | **Diamètre hydrodynamique (nm) - average** | ND | 88,64 |
| | **ZP (mV)_dans NaCl 0,15 mM** | ND | 36,9 |
| | **Stabilité** | ND | Ok |
| | **Complexation (%)** | ND | 100 |
| | **Efficacité d'extinction (%)** | ND | 49,34 |

**Tableau 5**

| | | **C1(comp.)** | **C2** | **C3** |
|---|---|---|---|---|
| **COURONNE** | **Lipide amphiphile : Lipoid** | S75-3 | S75-3 | S75-3 |
| | ***% wt Lipoid* / *gouttelette*** | 28,44 | 7,11 | 4,27 |
| | *% wt Lipoid* / *couronne sans PEG* | 100 | 25 | 15 |
| | *% wt Lipoid l couronne* | 70,24 | 17,56 | 10,54 |
| | *% mol. Lipoid l couronne* | 86,55 | 20,65 | 12,38 |
| | **Tensioactif cationique DOTAP** | X | DOTAP | DOTAP |
| | ***% wt DO TAP* / *gouttelette*** | 0 | 21,33 | 21,33 |
| | *% wt DOTAP* / *couronne sans PEG* | 0 | 75 | 75 |
| | *% wt DOTAP* / *couronne* | 0 | 52,68 | 52,68 |
| | ***% mol. DOTAP* / *couronne*** | **0** | **66,51** | **66,47** |
| | **Co-tensioactif** | Myrj S40 | Myrj S40 | Myrj S40 |
| | *%* ***wt_co-tensioactif* / *gouttelette*** | 12,05 | 12,05 | 12,05 |
| | *% wt (co-tensioactiof)* / *couronne* | 29,76 | 29,76 | 29,76 |
| | ***% mol. (co-tensioactif)* / *couronne*** | **13,45** | **12,84** | **12,83** |
| | **Lipide fusogène DOPE** | X | X | DOPE |
| | *%* ***wt DOPE* / *gouttelette*** | 0 | 0 | 2,84 |
| | *% wt DOPE l couronne sans PEG* | 0 | 0 | 10 |
| | *% wt DOPE l couronne* | 0 | 0 | 7,02 |
| | *% mol. DOPE l couronne* | 0 | 0 | 8,32 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 44,63 | 44,63 | 44,63 |
| | **Huile** | Huile de soja | Huile de soja | Huile de soja |
| | *% wt huile* / *coeur* | 25 | 25 | 25 |
| | ***% wt huile* / *gouttelette*** | 14,88 | 14,88 | 14,88 |
| | ***% wt coeur* / *gouttelette*** | | 59,51 | 59,51 |
| | ***% mol. coeur* / *gouttelette*** | | 65,85 | |
| | **Formulation (nombre de répétition)** | 4 | 4 | 3 |
| **RESULTAT** | **Diamètre hydrodynamique (nm) - average** | 153,03 | 162,2 | 168,9 |
| | **ZP (mV)_dans NaCl 0,15 mM** | -37,71 | 53,7 | 51,83 |
| | **Stabilité** | ok | ok | ok |
| | **Complexation (%)** | 0 | 100 | 100 |
| | **Efficacité d'extinction (%)** | 0 | 80,51 | 81,72 |

Dans les tableaux 1 à 5 :
% wt correspond à un pourcentage massique.
% mol. correspond à un pourcentage molaire.
ND (non déterminé) signifie que l'expérience n'a pas été réalisée
Les pourcentages « /gouttelette » représentent des pourcentages par rapport à l'ensemble (Lipoid/DOTAP/Myrj S40/ éventuel DOPE/Suppocire NB/Huile de soja).
Les pourcentages « /couronne » représentent des pourcentages par rapport à l'ensemble (Lipoid/DOTAP/Myrj S40/éventuel DOPE).
Les pourcentages « /couronne sans PEG » représentent des pourcentages par rapport à l'ensemble (Lipoid/DOTAP/éventuel DOPE).
Les pourcentages «/coeur» représentent des pourcentages par rapport à l'ensemble (Suppocire NB/Huile de soja).
Le Lipoid S75-3 comprend 65-75% de phosphatidylcholine. Les chaines aliphatiques des phospholipides sont majoritairement saturées (composition moyenne : 12-16 % de C16:0, 80-85% de C18:0, < 5 % de C18:1, < 2 % de C18:2).
Le Lipoid S75 comprend 65-75% de phosphatidylcholine. Les chaines aliphatiques des phospholipides sont majoritairement insaturées (composition moyenne: 17-20 % de C16:0, 2-5 % de C18:0, 8-12 % de C18:1, 58-65 % de C18:2, 4-6 % de C18:3).
Le Lipoid S100-3 comprend >94 % de phosphatidylcholine. Les chaines aliphatiques des phospholipides sont majoritairement saturées (composition moyenne : 12-16 % de C16:0, 85-88% de C18:0, < 2 % de C18:1, < 1 % de C18:2).

Les formulations A1, B1, B4 et C1 sont des exemples comparatifs, car elles ne comprennent pas de co-tensioactif cationique.
Leurs potentiels zêta sont négatifs.
La complexation de siARN n'a pas eu lieu en surface des gouttelettes, conformément à ce qui était attendu.

La formulation B9 est un exemple comparatif, car elle ne comprend pas de lipide amphiphile. L'émulsion n'a pas pu être préparée.

Le procédé de préparation suivant a été suivi :

### (i) Préparation de la phase huileuse :

L'huile de soja, la suppocire NC, le lipide amphiphile, le DOTAP, l'éventuel DOPE, ont été pesés puis mélangées avec du dichlorométhane avant d'être chauffés à 60°C afin d'obtenir une solution visqueuse homogène. Le dichlorométhane permet de favoriser la solubilisation. Les solvants ont ensuite évaporés sous vide.

### (ii) Préparation de la phase aqueuse :

Pendant la phase d'évaporation de l'éthanol, la phase aqueuse a été préparée. Dans un eppendorf de 5ml, le co-tensioactif, du glycérol et la solution aqueuse de PBS (NaCl 154 mM, pH 7,4) ont été mélangés puis dissous dans un bain à 75°C.

### (iii) Mélange des deux phases :

La phase huileuse était à environ 40°C (sous forme visqueuse) et la phase aqueuse à environ 70°C (en sortie du bain). La phase aqueuse a été versée dans la phase huileuse.

### (iv) Emulsification :

Le flacon contenant les deux phases a été fixé dans l'enceinte de sonication d'un sonificateur AV505® (Sonics, Newton, USA). Le protocole consistait en la réalisation de cycles de sonication (10 secondes d'activité toutes les 30 secondes) à une puissance de 100 W sur une période de 40 minutes.

### (v) Purification :

Les gouttelettes ainsi produites ont ensuite été purifiées par dialyse (seuil de coupure : 12 kDa, contre du NaCl 154 mM, sur une nuit) pour éliminer les composants lipidiques non-intégrés aux LNP. Finalement, la formulation a été stérilisée par filtration sur membrane de cellulose.

### • Taille des gouttelettes des formulations et potentiel zéta

### - Influence de la composition de la formulation

Les résultats des tableaux 1 à 5 montrent qu'une diminution de la proportion en co-tensioactif (Myrj S40) conduit à une augmentation du diamètre des gouttelettes.

### - Evolution dans le temps

L'évolution de la taille des gouttelettes (Tableau 6) et du potentiel zéta (tableau 7) des formulations ont été mesurées à 40°C (stabilité accélérée). Les formulations étaient conservées à 40°C entre deux mesures.

**Tableau 6 : Evolution de la taille des gouttelettes et index de polydispersité (PDI) mesurés par diffusion quasi-élastique de la lumière en fonction du temps**

| Jours | **B1** | **B4** | **B5** | **B6** | **B10** | **C1** | **C2** |
|---|---|---|---|---|---|---|---|
| **0** | 58,27 | 98,87 | 60,03 | 87,93 | 91,53 | 157,43 | 171,4 |
| **7** | 60,52 | 97,12 | 58,96 | 87,1 | 90,51 | 156,17 | 172,63 |
| **14** | 62,59 | 98,36 | 58,28 | 86,25 | 89,87 | 156,46 | 170,15 |
| **21** | 59,41 | 97,81 | 59,23 | 85,78 | 90,3 | 153,03 | 162,2 |
| **28** | 61 | 97,15 | 60,36 | 87,61 | 90,96 | 153,9 | 161,47 |

**Tableau 7 : Evolution du potentiel zéta des formulations en fonction du temps**

| Jours | **B1** | **B4** | **B5** | **B6** | **B10** | **C1** | **C2** |
|---|---|---|---|---|---|---|---|
| **0** | -21,3 | -21,16 | 24,03 | 34,13 | 34,97 | -40,27 | 57,33 |
| **7** | -25,6 | -21,17 | 28,23 | 31,77 | 34,73 | -43,13 | 56,77 |
| **14** | -24,73 | -21,03 | 29,45 | 28,4 | 33,47 | -42,33 | 55,33 |
| **21** | -21,8 | -20,47 | 28,63 | 34,3 | 33,07 | -38,1 | 53,9 |
| **28** | -18,93 | -19,83 | 27,5 | 33 | 31,23 | -39,03 | 51,6 |

Il a été observé que la taille des gouttelettes et le potentiel zéta de formulations selon l'invention conservées à 40°C pendant 300 jours n'évoluent pas.

Ces résultats montrent que les formulations selon l'invention sont stables dans le temps.

### • Complexation avec des siARN : Préparation de formulations « finales » comprenant des séquences nucléotiaues siARN.

La procédure générale suivante a été suivie :

La complexation consiste en un simple mélange des formulations préparées ci-dessus et d'une solution de siARN, le tout dans un tampon. Le choix du tampon est fonction de l'application envisagée : pour une étude *in vitro,* le milieu de culture optimisé pour les étapes de transfection, OptiMEM, a été utilisé. Pour une étude de complexation, du tampon Hepes 5 mM a été utilisé.

Une quantité de 0,5 µg de siARN (GFP-22 siRNA rhodamine (catalogue n°1022176) (Qiagen) ou siGFP (Sigma)) a été utilisée (25 µg/mL dans 20 µL).

Le mélange a été agité durant 30 minutes à 600 rpm, ceci à température ambiante (environ 25°C).

La complexation a été visualisée à travers deux outils :
- L'électrophorèse sur gel d'agarose qui permet d'observer la migration des siARN. S'il y a une bonne complexation, alors les gouttelettes comprenant les siARN complexés seront plus lourdes que les siARN libres et seront visualisés dans les puits. Si la complexation est moins importante, des siARN libres migrent à une autre position.
- En DLS en observant l'impact de la complexation sur le diamètre hydrodynamique. Plus la complexation est efficace, plus le profil s'oriente vers une distribution monomodale.

La quantité de formulation nécessaire pour avoir un rendement de complexation quantitatif des siARN a été optimisée.

En pratique, les charges négatives apportées par les siARN sont compensées par les charges positives de la formulation (c'est-à-dire les charges positives du tensioactif cationiques DOTAP).

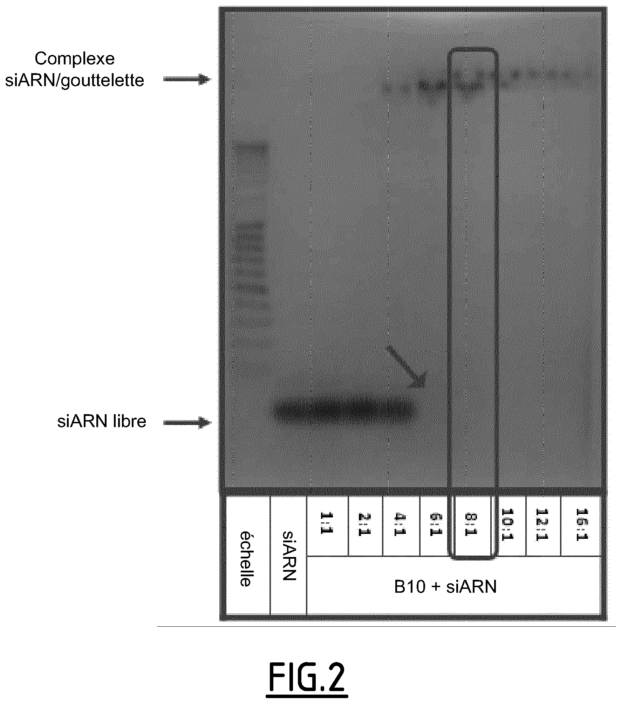

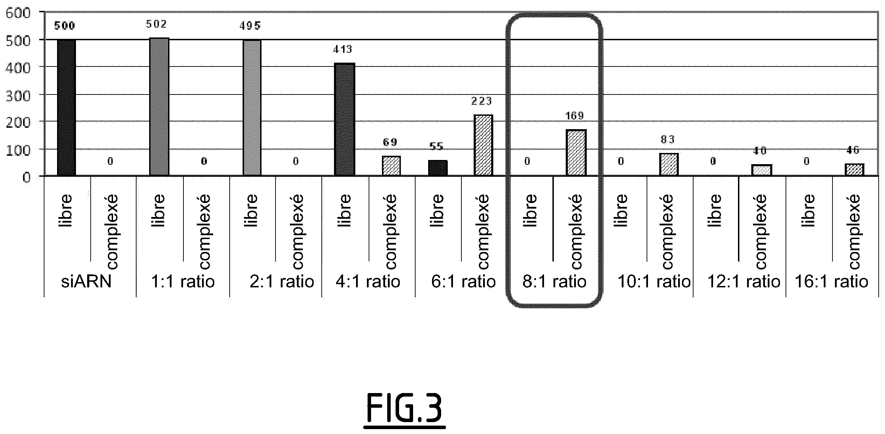

Typiquement, lorsque le seul tensioactif cationique de la formulation est du DOTAP (qui ne comprend qu'une seule charge positive), un rendement quantitatif de complexation est obtenu lorsque le rapport quantité de charges positives dues au tensioactif cationique dans la formulation « prémix » sur la quantité de charge négative apportée par les siARN est supérieur à 8/1, comme illustré sur les figures 2 et 3.

La figure 2 représente un gel d'électrophorèse révélé à l'UV réalisé après complexation de siARN avec la formulation B10 avec des concentrations précisées au tableau 8, en mélangeant une solution de siARN et la formulation dans un tampon Hepes 5 mM. Avant dépôt sur gel d'agarose 1,5 %, 2 µL de tampon de charge ont été ajoutés aux essais. Après 1h30 d'électrophorèse à 100 V, le gel a été plongé dans du GeIRed 3X. Enfin, une révélation UV a été effectuée.

**Tableau 8**

| | rapport quantité de charges positives dues au tensioactif cationique dans la formulation « prémix » sur quantité de charge négative apportée par les siARN | 1/1 | 2/1 | 4/1 | 6/1 | 8/1 | 10/1 | 12/1 | 16/1 |
|---|---|---|---|---|---|---|---|---|---|
| Concentration en siARN (µg/mL) | 25 | | | | | | | | |
| Concentration en DOTAP (µg/mL) | 0 | 25 | 50 | 100 | 150 | 200 | 250 | 300 | 400 |

La figure 3 donne les résultats obtenus par traitement des données de l'électrophorèse sur le logiciel ImageJ des mêmes expériences.

Les figures 2 et 3 montrent que pour un rapport quantité de charges positives dues au tensioactif cationique dans la formulation « prémix » sur la quantité de charge négative apportée par les siARN supérieur à 8/1, il n'y a plus de siARN libre dans le milieu et que le siARN a été complètement complexé.

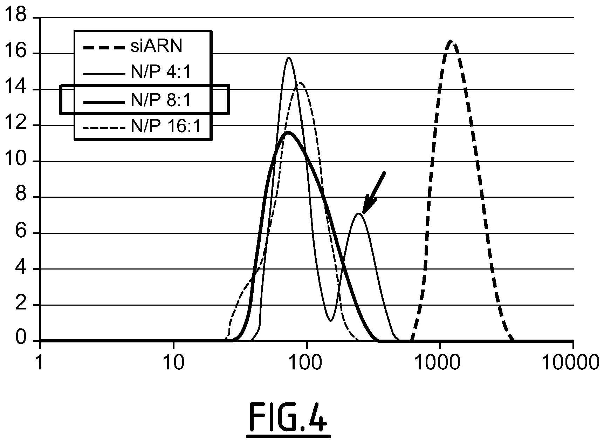

La figure 4 représente l'intensité obtenu sur un appareil ZetaSizer Malvern en fonction du diamètre hydrodynamique en nm pour le siARN libre (comparaison) et pour trois formulations selon l'invention obtenues par complexation avec un rapport quantité de charges positives dues au tensioactif cationique dans la formulation « prémix » sur la quantité de charge négative apportée par les siARN de 4/1, de 8/1 ou de 16/1.

Le diamètre est plus important pour le siARN libre (comparaison).

Avec un rapport quantité de charges positives dues au tensioactif cationique dans la formulation « prémix » sur la quantité de charge négative apportée par les siARN de 4/1, on a observé 2 populations : un complexe siARN/gouttelette autour de 100 nm et une population de taille supérieure représentant les siARN sous forme libre (flèche).

Avec les rapports quantité de charges positives dues au tensioactif cationique dans la formulation « prémix » sur la quantité de charge négative apportée par les siARN de 8/1 et 16/1, une seule population représentant le complexe siARN/ gouttelette a été observée.

Ces résultats montrent également qu'une complexation quantitative (100%) des siARN sur les gouttelettes est permise.

L'étape de complexation a été réalisée avec diverses formulations.

A titre comparatif, un test de complexation a été réalisé avec une formulation exempte de tensioactif cationique : la formulation B1 décrite ci-dessus. Comme attendu, la complexation du siARN n'a pas eu lieu et le siARN est resté sous forme libre.

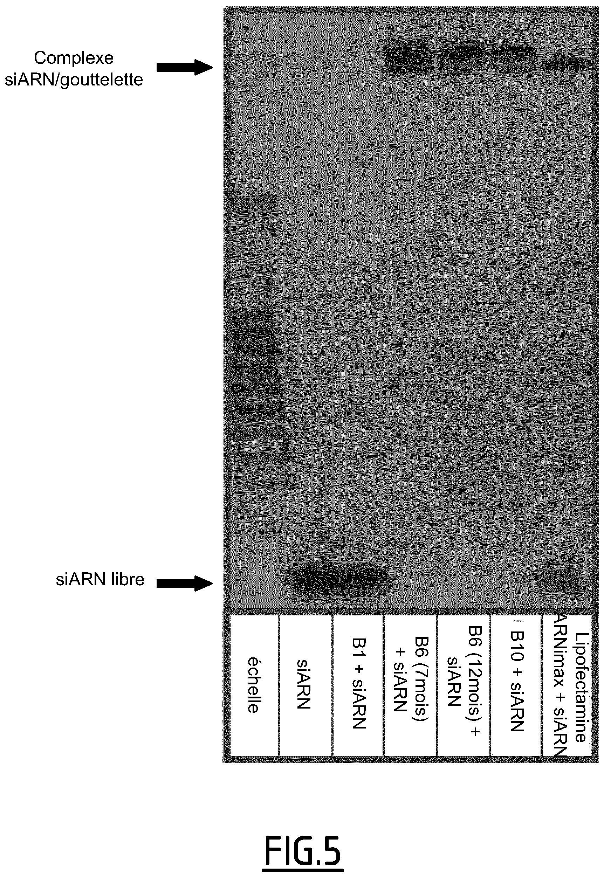

La figure 5 représente un gel d'électrophorèse révélé à l'UV avec, de gauche à droite :
- l'échelle,
- le siARN libre (témoin)
puis des migrations obtenues par complexation de siARN :
- avec la formulation B1 (exemple comparatif) (pas de complexation, les siARN sont libres),
- avec la formulation B6 (prémix formulé 6 mois auparavant) avec un rapport quantité de charges positives dues au tensioactif cationique dans la formulation « prémix » sur la quantité de charge négative apportée par les siARN de 8/1 (complexation quantitative),
- avec la formulation B6 (prémix formulé 12 mois auparavant) avec un rapport quantité de charges positives dues au tensioactif cationique dans la formulation « prémix » sur la quantité de charge négative apportée par les siARN de 8/1, la formulation ayant été conservée 7 mois à température ambiante préalablement à la complexation (complexation quantitative),
- avec la formulation B10 avec un rapport quantité de charges positives dues au tensioactif cationique dans la formulation « prémix » sur la quantité de charge négative apportée par les siARN de 8/1 (complexation quantitative),
- avec de la lipofectamine (exemple comparatif) (complexation incomplète).

La complexation de siARN est quantitative lorsque la formulation B6 ou B10 ont été utilisées. Le stockage de la formulation à température ambiante avant sa complexation avec le siARN n'a pas d'influence sur le rendement de complexation, qui reste quantitatif, ce qui montre la stabilité des formulations utilisées.

Le rendement est quantitatif que la formulation utilisée comprennent ou non du DOPE.

Avec l'agent de transfection commercial Lipofectamine RNAimax, plus de 60 % des siARN ont été retrouvés sous forme libre. Ceci implique que les formulations de nanoémulsions utilisées dans l'invention offrent un meilleur rendement de complexation que la Lipofectamine.

Enfin, une cinétique de relargage des siARN du complexe siARN/formulation B10 préparé ci-dessus a été effectuée pour observer l'évolution de la complexation au cours du temps et est illustrée en figure 6. Jusqu'à 3 heures après complexation, les siARN ne sont pas retrouvés sous forme libre. A 6 heures, les siARN commencent à être relargués. Le complexe est donc stable au moins trois heures.

### • Transfection in vitro

Des tests de transfection ont été effectués sur différentes lignées cellulaires surexprimant la Green Fluorescent Protein (GFP, Protéine de fluorescence verte) en apportant un siARN inhibant spécifiquement une séquence de l'ARN messager de cette protéine (GFP-22 siRNA rhodamine (catalog n°1022176) (Qiagen)).

La transfection a été effectuée avec une concentration finale en siARN de 100 nM. Ainsi, des cellules exprimant la GFP ont été ensemencées dans des plaques 12 puits (25 000 cellules/puits) puits traitées avec les complexes siARN/Formulations (B1, B6, B6 7 mois après sa préparation ou B10) obtenus ci-dessus. Les cellules sont ensuite incubées 72 heures à 37 °C, puis récupérées pour analyse de l'intensité de fluorescence au cytomètre en flux pour déterminer l'efficacité de la formulation en tant qu'agent de transfection. La délivrance active de siARN inhibant spécifiquement l'expression de la protéine GFP induit une diminution de la fluorescence apportée par cette protéine.

L'agent de transfection commercial, la Lipofectamine RNAimax a été utilisé pour comparaison.

La figure 7 représente la diminution de l'intensité de fluorescence FITC en % en transfectant les lignées cellulaires avec :
- de la Lipofectamine RNAimax,
- le complexe siARN/formulation B1,
- avec le complexe siARN/formulation B6, la formulation ayant été conservée 7 mois à température ambiante préalablement à la complexation,
- avec le complexe siARN/formulation B6, la formulation ayant été conservée 12 mois à température ambiante préalablement à la complexation,
- avec le complexe siARN/formulation B10,
- avec un complexe siARN / formulation de liposomes cationiques comprenant du DOTAP (58% wt), du DOPE (18% wt), du cholestérol (2% wt) et du DSPE-PEG3000 (22% wt)) (comparatif).

Ainsi, une diminution de la fluorescence de 33 à 50% est observée avec les formulations selon l'invention testées. Les formulations selon l'invention permettent donc une délivrance active de siARN induisant une extinction relative de l'expression du gène de la GFP.

De plus, en incorporant du DOPE dans les formulations, une plus importante extinction de fluorescence est visible, le DOPE favorisant l'échappement endosomal.

Aucune diminution de la fluorescence n'a été observée avec le complexe ssiARN / formulation de liposomes cationiques, ce qui pourrait par exemple s'expliquer par une mauvaise stabilité des liposomes dans le milieu de culture, un mauvais rendement de complexation et/ou une mauvaise rétention des siARN par les liposomes après complexation.

Enfin, de tels résultats sur la délivrance active de siARN médiée par les formulations selon l'invention ont été reproduits sur 3 lignées cellulaires exprimant la GFP : U2OS, PC3 et Hela, comme illustré en figure 8.

### • Complexation avec un microARN synthétique : Préparation de formulations « finales » comprenant des séquences nucléotiaues de microARN synthétique (mimic).

La procédure générale suivante a été suivie :

La complexation consiste en un simple mélange de la formulation premix A3 préparée ci-dessus et d'une solution de microARN (miRIDIAN Mimic Human has-miR612 (ThermoScientific REF#C-300937-01 Lot n°130611)), le tout dans un tampon. Le choix du tampon est fonction de l'application envisagée : pour une étude *in vitro,* le milieu de culture optimisé pour les étapes de transfection, OptiMEM®, a été utilisé. Pour une étude de complexation, du tampon HEPES 5 mM a été utilisé.

Une quantité de 0,5 µg de microARN (miRIDIAN Mimic Human has-miR612 (ThermoScientific REF#C-300937-01 Lot n°130611) a été utilisée.

Le mélange a été agité durant 30 minutes à 600 rpm, ceci à température ambiante (environ 25°C).

La complexation a été visualisée par révélation d'un gel d'agarose obtenu par électrophorèse, qui permet d'observer la migration des microARN. S'il y a une bonne complexation, alors les gouttelettes comprenant les microARN complexés sont plus lourdes que les microARN libres et seront visualisés dans les puits. Si la complexation est moins importante, des microARN libres migrent à une autre position.

La quantité de formulation premix A3 nécessaire pour avoir un rendement de complexation quantitatif des microARN a été optimisée.

En pratique, les charges négatives apportées par les microARN sont compensées par les charges positives de la formulation premix (c'est-à-dire les charges positives du tensioactif cationiques DOTAP). Typiquement, lorsque le seul tensioactif cationique de la formulation premix est du DOTAP (qui ne comprend qu'une seule charge positive), un rendement quantitatif de complexation est obtenu lorsque le rapport quantité de charges positives dues au tensioactif cationique dans la formulation « prémix » sur la quantité de charge négative apportée par les microARN (rapport N/P) est supérieur à 8/1, comme précédemment avec les siARN.

La figure 9 représente un gel d'électrophorèse révélé à l'UV réalisé après complexation de microARN avec la formulation A3 avec des concentrations précisées au tableau 9, en mélangeant une solution de microARN et la formulation premix A3 dans un tampon HEPES 5 mM. Avant dépôt sur gel d'agarose 1,5 %, 2 µL de tampon de charge ont été ajoutés aux essais. Après 1h30 d'électrophorèse à 100 V, le gel a été plongé dans du GelRed 3X. Enfin, une révélation UV a été effectuée.

**Tableau 9**

| | rapport quantité de charges positives dues au tensioactif cationique dans la formulation « prémix » sur quantité de charges négatives apportées par les microARN | 1/1 | 2/1 | 4/1 | 6/1 | 8/1 | 10/1 | 12/1 | 16/1 |
|---|---|---|---|---|---|---|---|---|---|
| Concentration en microARN (µg/mL) | 25 | | | | | | | | |
| Concentration en DOTAP (µg/mL) | 0 | 25 | 50 | 100 | 150 | 200 | 250 | 300 | 400 |

La figure 9 montre que pour un rapport quantité de charges positives dues au tensioactif cationique dans la formulation « prémix » sur la quantité de charge négative apportée par les microARN supérieur à 8/1, il n'y a plus de microARN libre dans le milieu et que le microARN a été complètement complexé, comme illustré dans les figures 2 et 3 avec les siARN.

## Revendications

1. Formulation sous forme de nanoémulsion, comprenant une phase aqueuse continue et au moins une phase dispersée, et comprenant :
- au moins 5% molaire de lipide amphiphile,
- de 15 à 70% molaire d'au moins un tensioactif cationique comprenant :
- au moins un groupe lipophile choisi parmi :
- un groupe R ou R-(C=O)-, où R représente une chaîne hydrocarbonée linéaire comprenant de 11 à 23 atomes de carbone,
- un ester ou un amide d'acides gras comprenant de 12 à 24 atomes de carbone et de phosphatidyléthanolamine, et
- un poly(oxyde de propylène), et
- au moins un groupe hydrophile comprenant au moins un groupe cationique choisi parmi :
- un groupe alkyle linéaire ou ramifié comprenant de 1 à 12 atomes de carbone et interrompu et/ou substitué par au moins un groupe cationique, et
- un groupe polymérique hydrophile comprenant au moins un groupe cationique, et
- de 10% à 55% molaire d'un co-tensioactif comprenant au moins une chaîne poly(oxyde d'éthylène) comprenant au moins 25 unités d'oxyde d'éthylène,
- un lipide solubilisant,
- éventuellement un lipide fusogène,
- éventuellement une huile,
- éventuellement un agent d'imagerie, et
- éventuellement un agent thérapeutique lipophile,
où les pourcentages molaires de lipide amphiphile, de tensioactif cationique et de co-tensioactif sont par rapport à l'ensemble (lipide amphiphile / tensioactif cationique / co-tensioactif / éventuel lipide fusogène), et
où la proportion molaire de l'ensemble (lipide solubilisant / éventuelle huile / éventuel agent d'imagerie / éventuel agent thérapeutique lipophile) par rapport à la phase dispersée est de 10 à 80%.

2. Formulation selon la revendication 1, dans laquelle la proportion molaire de l'ensemble (lipide solubilisant / éventuelle huile / éventuel agent d'imagerie / éventuel agent thérapeutique lipophile) par rapport à la phase dispersée est de 25 à 75%, de préférence de 33,35 à 73,99%.

3. Formulation selon la revendication 1 ou 2, dans laquelle le tensioactif cationique est choisi parmi :
- le *N*[1-(2,3-dioléyloxy) propyl]-*N,N,N*-triméthylammonium,
- le 1,2-dioleyl-3-trimethylamonium-propane,
- le *N*-(2-hydroxyethyl)-*N,N*-dimethyl-2,3-bis(tetradecyloxy-1-propananium),
- le 1-[2-(oleoyloxy)ethyl]-2-oleyl-3-(2-hydroxyethyl)imidazolinium, et
- le dioctadecylamidoglycylspermine.

4. Formulation selon l'une quelconque des revendications 1 à 3, comprenant un lipide fusogène, tel que la 1,2-Dioléoyl-sn-Glycéro-3-Phosphoéthanolamine.

5. Formulation selon l'une quelconque des revendications 1 à 4, dans laquelle le lipide amphiphile est un phospholipide.

6. Formulation selon l'une quelconque des revendications 1 à 5, comprenant un agent d'imagerie.

7. Formulation selon l'une quelconque des revendications 1 à 6, comprenant un agent thérapeutique.

8. Formulation selon l'une quelconque des revendications 1 à 7, comprenant un co-tensioactif greffé avec une molécule d'intérêt, de préférence un ligand biologique de ciblage.

9. Formulation selon l'une quelconque des revendications 1 à 8, comprenant une séquence nucléotidique mono ou double brin, comprenant moins de 200 bases pour une séquence nucléotidique mono brin ou moins de 200 paires de base pour une séquence nucléotidique double brin, et susceptible de moduler des mécanismes endogènes d'ARN interférence.

10. Formulation selon la revendication 9, où ladite séquence nucléotidique est choisie parmi :
- un petit ARN interférent,
- un acide nucléique bloqué, et
- un microARN synthétique.

11. Formulation selon la revendication 10, où ladite séquence nucléotidique est un petit ARN interférent.

12. Procédé de préparation d'une formulation selon l'une quelconque des revendications 9 à 11, comprenant les étapes suivantes :
(i) préparer une phase huileuse comprenant un lipide solubilisant, un lipide amphiphile, le tensioactif cationique;
(ii) préparer une phase aqueuse comprenant le co-tensioactif;
(iii) disperser la phase huileuse dans la phase aqueuse sous l'action d'un cisaillement suffisant pour former une formulation sous forme de nanoémulsion telle que définie dans l'une quelconque des revendications 1 à 8; puis
(iv) ajouter une séquence nucléotidique mono ou double brin, comprenant moins de 200 bases pour une séquence nucléotidique mono brin ou moins de 200 paires de base pour une séquence nucléotidique double brin, et susceptible de moduler des mécanismes endogènes d'ARN interférence à la formulation sous forme de nanoémulsion telle que définie dans l'une quelconque des revendications 1 à 8, puis
(v) récupérer la formulation ainsi formée.

13. Méthode d'introduction in vitro d'une séquence nucléotidique mono ou double brin, comprenant moins de 200 bases pour une séquence nucléotidique mono brin ou moins de 200 paires de base pour une séquence nucléotidique double brin, et susceptible de moduler des mécanismes endogènes d'ARN interférence dans une cellule eucaryote, comprenant la mise en contact de la cellule eucaryote avec une formulation selon l'une quelconque des revendications 9 à 11.

14. Formulation selon l'une quelconque des revendications 9 à 11 pour son utilisation pour la prévention et/ou le traitement d'une maladie.

15. Kit comprenant la formulation sous forme de nanoémulsion telle que définie selon l'une quelconque des revendications 1 à 8, et séparément, au moins une séquence nucléotidique mono ou double brin, comprenant moins de 200 bases pour une séquence nucléotidique mono brin ou moins de 200 paires de base pour une séquence nucléotidique double brin, et susceptible de moduler des mécanismes endogènes d'ARN interférence.

## Patentansprüche

1. Formulierung in Form einer Nanoemulsion, umfassend eine kontinuierliche wässrige Phase und mindestens eine dispergierte Phase, und umfassend:
- mindestens 5 Mol.-% amphiphiles Lipid
- von 15 bis 70 Mol.-% mindestens eines kationischen Tensids, umfassend:
- mindestens eine lipophile Gruppe ausgewählt aus:
- einer Gruppe (R) oder R-(C=O)-, wobei R eine lineare Kohlenwasserstoffkette, umfassend 11 bis 23 Kohlenstoffatome, darstellt,
- ein Ester oder ein Amid einer Fettsäure, umfassend 12 bis 24 Kohlenstoffatome und Phosphatidylethanolamin, und
- ein Poly(propylenoxid), und
- mindestens eine hydrophile Gruppe, die mindestens eine kationische Gruppe umfasst, ausgewählt aus:
- eine lineare oder verzweigte Alkylgruppe, umfassend 1 bis 12 Kohlenstoffatome und unterbrochen und/oder substituiert durch mindestens eine kationische Gruppe, und
- eine hydrophile Polymergruppe, die mindestens eine kationische Gruppe umfasst, und
- 10 bis 55 Mol.-% eines Co-Tensids, umfassend mindestens eine Poly(ethylenoxyd)kette, die mindestens 25 Einheiten an Ethylenoxid umfasst,
- ein solubilisierendes Lipid,
- gegebenenfalls ein fusogenes Lipid,
- gegebenenfalls Öl,
- gegebenenfalls ein Kontrastmittel, und
- gegebenenfalls ein lipophiles Therapeutikum,
wobei die Molprozente an amphiphilem Lipid, kationischem Tensid und Co-Tensid sich auf die Gesamtheit (amphiphiles Lipid / kationisches Tensid / Co-Tensid / gegebenenfalls fusogenes Lipid) beziehen, und
wobei das Molverhältnis der Gesamtheit (solubilisierendes Lipid / gegebenenfalls Öl / gegebenenfalls Kontrastmittel / gegebenenfalls lipophiles Therapeutikum) in Bezug auf die dispergierte Phase von 10 bis 80% beträgt.

2. Formulierung nach Anspruch 1, bei der das Molverhältnis der Gesamtheit (solubilisierendes Lipid / gegebenenfalls Öl / gegebenenfalls Kontrastmittel / gegebenenfalls lipophiles Therapeutikum) in Bezug auf die dispergierte Phase von 25 bis 75% ist, vorzugsweise von 33,35 bis 73,99% ist.

3. Formulierung nach Anspruch 1 oder 2, bei der das kationische Tensid ausgewählt ist aus:
- N-[1-(2,3-Dioleyloxy)propyl]-N,N,N-Trimethylammonium,
- 1,2-Dioleyl-3-Trimethylammonium-Propan,
- N-(2-Hydroxyethyl)-N,N-Dimethyl-2,3-bis(Tetradecyloxy-1-Propan),
- 1-[2-(Oleoyloxy)ethyl]-2-Oleyl-3-(2-Hydroxyethyl)imidazolin, und
- Dioctadecylamidoglycylspermin.

4. Formulierung nach einem beliebigen der Ansprüche 1 bis 3, aufweisend ein Fusogenlipid, wie 1,2-Dioleoyl-sn-Glycerin-3-Phosphoethanolamin.

5. Formulierung nach einem beliebigen der Ansprüche 1 bis 4, in dem das amphiphile Lipid ein Phospholipid ist.

6. Formulierung nach einem beliebigen der Ansprüche 1 bis 5, ein Kontrastmittel umfassend.

7. Formulierung nach einem beliebigen der Ansprüche 1 bis 6, ein Therapeutikum umfassend.

8. Formulierung nach einem beliebigen der Ansprüche 1 bis 7, ein Co-Tensid, gepfropft mit einem interessierenden Molekül, vorzugsweise einem biologischen Targetligand, umfassend.

9. Formulierung nach einem beliebigen der Ansprüche 1 bis 8, eine einzel- oder doppelsträngige Nucleotidsequenz, die weniger als 200 Basen einer einzelsträngigen Nucleotidsequenz oder weniger als 200 Basenpaare einer doppelsträngigen Nucleotidsequenz umfasst, und die geeignet ist, endogene Mechanismen der RNA-Interferenz zu modulieren.

10. Formulierung nach Anspruch 9, in der die Nucleotidsequenz ausgewählt ist aus:
- eine kleine interferierende RNA,
- eine blockierte Nucleinsäure, und
- eine synthetische Mikro-RNA.

11. Formulierung nach Anspruch 10, bei der die Nucleotidsequenz eine kleine interferierende RNA ist.

12. Verfahren zur Herstellung einer Formulierung nach einem beliebigen der Ansprüche 9 bis 11, folgende Schritte umfassend:
(i) Aufbereiten einer Ölphase, die ein solubilisierendes Lipid, ein amphiphiles Lipid, das kationische Tensid, enthält;
(ii) Aufbereiten einer wässrigen Phase, die das Co-Tensid umfasst;
(iii) Dispergieren der Ölphase in der wässrigen Phase unter Scherwirkung, die ausreichend ist, um eine Formulierung in Form einer Nanoemulsion zu bilden, wie sie in einem beliebigen der Ansprüche 1 bis 8 definiert ist; dann
(iv) Hinzufügen einer einzelsträngigen oder doppelsträngigen Nucleotidsequenz, die weniger als 200 Basen einer einsträngigen Nucleotidsequenz oder weniger als 200 Basenpaare einer doppelsträngigen Nucleotidsequenz umfasst, und geeignet ist, endogene Mechanismen der RNA-Interferenz zu modulieren, zu der Formulierung in Form der Nanoemulsion, wie sie in einem beliebigen der Ansprüche 1 bis 8 definiert ist, dann
(v) Rückgewinnen der so gebildeten Formulierung.

13. Verfahren zum Einführen in vitro einer einsträngigen oder doppelsträngigen Nucleotidsequenz, die weniger als 200 Basen einer einsträngigen Nucleotidsequenz oder weniger als 200 Basenpaare einer doppelsträngigen Nucleotidsequenz umfasst, und die geeignet ist, in einer eukaryotische Zelle endogene Mechanismen der RNA-Interferenz zu modulieren, umfassend das Inkontaktbringen der eukaryotischen Zelle mit der Formulierung nach einem beliebigen der Ansprüche 9 bis 11.

14. Formulierung nach einem beliebigen der Ansprüche 9 bis 11 zur Verwendung in der Prävention und/oder in der Behandlung einer Krankheit.

15. Kit, die Formulierung in Form der Nanoemulsion umfassend, die gemäß einem beliebigen der Ansprüche 1 bis 8 definiert ist, und getrennt mindestens eine einsträngige oder doppelsträngige Nucleotidsequenz, die weniger als 200 Basen einer einsträngigen Nucleotidsequenz oder weniger als 200 Basenpaare einer doppelsträngigen Nucleotidsequenz enthält und geeignet ist, endogene Mechanismen der RNA-Interferenz zu modulieren.

## Claims

1. A formulation in nanoemulsion form comprising a continuous aqueous phase and a least one dispersed phase, and comprising:
- at least 5 mole % of amphiphilic lipid;
- 15 to 70 mole % of at least one cationic surfactant comprising:
- at least one lipophilic group selected from among:
- an R or R-(C=O)- group, where R is a linear hydrocarbon chain having 11 to 23 carbon atoms,
- an ester or amide of fatty acids having 12 to 24 carbon atoms and phosphatidylethanolamine, and
- a poly(propylene oxide), and
- at least one hydrophilic group comprising at least one cationic group selected from among:
- a linear or branched alkyl group having 1 to 12 carbon atoms and interrupted and/or substituted by at least one cationic group; and
- a hydrophilic polymeric group comprising at least one cationic group; and
- 10 % to 55 mole % of a co-surfactant comprising at least one poly(ethylene oxide) chain comprising at least 25 ethylene oxide units;
- a solubilising lipid,
- optionally a helper lipid,
- optionally an oil,
- optionally an imaging agent, and
- optionally a lipophilic therapeutic agent,
wherein the mole percentages of amphiphilic lipid, cationic surfactant and co-surfactant are relative to the whole (amphiphilic lipid / cationic surfactant / co-surfactant / optional helper lipid), and
wherein the mole proportion of the whole (solubilising lipid / optional oil / optional imaging agent / optional lipophilic therapeutic agent) relative to the dispersed phase is 10 to 80 %.

2. The formulation according to claim 1 wherein the mole proportion of the whole (solubilising lipid / optional oil / optional imaging agent / optional lipophilic therapeutic agent) relative to the dispersed phase is 25 to 75%, preferably 33.35 to 73.99%.

3. The formulation according to claim 1 or 2, wherein the cationic surfactant is selected from among:
- *N*[1-(2,3-dioleyloxy) propyl]-*N,N,N*-trimethylammonium,
- 1,2-dioleyl-3-trimethylamonium-propane,
- *N*-(2-hydroxyethyl)-*N,N*-dimethyl-2,3-bis(tetradecyloxy-1-propananium),
- 1-[2-(oleoyloxy)ethyl]-2-oleyl-3-(2-hydroxyethyl)imidazolinium, and
- dioctadecylamidoglycylspermine.

4. The formulation according to any of claims 1 to 3 comprising a helper lipid such as 1,2-Dioleoyl-sn-Glycero-3-Phosphoethanolamine.

5. The formulation according to any of claims 1 to 4 wherein the amphiphilic lipid is a phospholipid.

6. The formulation according to any of claims 1 to 5 comprising an imaging agent.

7. The formulation according to any of claims 1 to 6 comprising a therapeutic agent.

8. The formulation according to any of claims 1 to 7 comprising a co-surfactant grafted with a molecule of interest, preferably a targeting biological ligand.

9. The formulation according to any of claims 1 to 8 comprising a single or double strand nucleotide sequence, comprising fewer than 200 bases for a single strand nucleotide sequences or fewer than 200 base pairs for a double strand nucleotide sequence, able to modulate endogenous mechanisms of RNA interference.

10. The formulation according to claim 9 wherein the said nucleotide sequence is selected from among:
- small interfering RNA;
- locked nucleic acid; and
- synthetic microRNA.

11. The formulation according to claim 10 wherein the said nucleotide sequence is small interfering RNA.

12. A method for preparing a formulation according to any of claims 8 to 10 comprising the following steps:
(i) preparing an oil phase comprising a solubilising lipid, an amphiphilic lipid, the cationic surfactant;
(ii) preparing an aqueous phase comprising the co-surfactant;
(iii) dispersing the oil phase in the aqueous phase under sufficient shear action to obtain a formulation in nanoemulsion form such as defined in any of claims 1 to 7; then
(iv) adding a single or double strand nucleotide sequence, comprising fewer than 200 bases for a single strand nucleotide sequence or fewer than 200 base pairs for a double strand nucleotide sequence, able to modulate endogenous mechanisms of RNA interference, to the formulation in emulsion form such as defined in any of claims 1 to 7, then
(v) recovering the formulation thus formed.

13. A method for *in vitro* insertion into a eukaryote cell of a single or double strand nucleotide sequence, comprising fewer than 200 bases for a single nucleotide sequence or fewer than 200 base pairs for a double strand nucleotide sequence, able to modulate endogenous mechanisms of RNA interference, comprising the contacting of the eukaryote cell with a formulation according to any of claims 9 to 11.

14. The formulation according to any of claims 9 to 11 for use thereof to prevent and/or treat a disease.

15. A kit comprising the formulation in nanoemulsion form such as defined in any of claims 1 to 8 and, separately, at least one single or double strand nucleotide sequence comprising fewer than 200 bases for a single strand nucleotide sequence or fewer than 200 base pairs for a double strand nucleotide sequence, that is able to modulate endogenous mechanisms of RNA interference.
